# EUROPÄISCHE PATENTSCHRIFT

(11) **EP 1 487 834 B1**
(45) Veröffentlichungstag und Bekanntmachung des Hinweises auf die Patenterteilung: **25.04.2007**
(21) Anmeldenummer: 03708168.4
(22) Anmeldetag: 03.03.2003
(51) Int. Cl.: C07D 453/02, A61K 31/439, A61P 25/28

(54) **AZA-BICYCLISCHE N-BIARYLAMIDE MIT AFFINITÄT ZUM ALPHA7 NIKOTINISCHEN ACETYLCHOLIN-REZEPTOR**
AZA-BICYCLIC N-BIARYLAMIDES WITH AFFINITY FOR THE ALPHA-7 NICOTINIC ACETYLCHOLINE RECEPTOR
N-BIARYLAMIDES AZA-BICYCLIQUES AYANT UNE AFFINITE AU RECEPTEUR D'ACETYLE-CHOLINE ALPHA7 NICOTINIQUE

(30) Priorität: 15.03.2002 DE 10211415
(43) Veröffentlichungstag der Anmeldung: 22.12.2004
(73) Patentinhaber: Bayer HealthCare AG, 51368 Leverkusen (DE)
(72) Erfinder: LUITHLE, Joachim, 42489 Wülfrath (DE); BÖSS, Frank-Gerhard, Berkshire SL5 7DF (GB); ERB, Christina, 65380 Kriftel (DE); SCHNITZLER, Katrin, 63517 Rodenbach (DE); FLESSNER, Timo, 42113 Wuppertal (DE); VAN KAMPEN, Marja, 63263 Neu-Isenburg (DE); METHFESSEL, Christoph, 42327 Wuppertal (DE); HAFNER, Frank-Thorsten, 42115 Wuppertal (DE)
(86) Internationale Anmeldenummer: PCT/EP2003/002153
(87) Internationale Veröffentlichungsnummer: WO 2003/078431

(56) Entgegenhaltungen:
- US-A- 5 998 429

## Beschreibung

Die Erfindung betrifft neue bicyclische N-Biarylamide, Verfahren zu ihrer Herstellung sowie ihre Verwendung zur Herstellung von Arzneimitteln zur Behandlung und/oder Prophylaxe von Krankheiten und zur Verbesserung der Wahrnehmung, Konzentrationsleistung, Lernleistung und/oder Gedächtnisleistung.

Nikotinische Acetylcholin-Rezeptoren (nAChR) bilden eine große Familie von Ionenkanälen, die durch den körpereigenen Botenstoff Acetylcholin aktiviert werden (Galzi und Changeux, *Neuropharmacol.* **1995,** *34,* 563-582). Ein funktioneller nAChR besteht aus fünf Untereinheiten, die unterschiedlich (bestimmte Kombinationen von α1-9 und β1-4,γ,δ,ε-Untereinheiten) oder identisch (α7-9) sein können. Dies führt zur Bildung einer Vielfalt von Subtypen, die eine unterschiedliche Verteilung in der Muskulatur, dem Nervensystem und anderen Organen zeigen (McGehee und Role, *Annu. Rev. Physiol*., **1995,** *57*, 521-546). Aktivierung von nAChR führt zum Einstrom von Kationen in die Zelle und zur Stimulation von Nerven- oder Muskelzellen. Selektive Aktivierung einzelner nAChR-Subtypen beschränkt diese Stimulation auf die Zelltypen, die den entsprechenden Subtyp besitzen und kann so unerwünschte Nebeneffekte wie z.B. die Stimulierung von nAChR in der Muskulatur vermeiden. Klinische Experimente mit Nikötin und Experimente in verschiedenen Tiermodellen weisen auf eine Rolle von zentralen nikotinischen Acetylcholin-Rezeptoren bei Lern- und Gedächtnisvorgängen hin (z.B. Rezvani and Levin, *Biol. Psychiatry* **2001**, *49*, 258-267). Nikotinische Acetylcholinrezeptoren des alpha7-Subtyps (α7-nAChR) haben eine besonders hohe Konzentration in für Lernen und Gedächtnis wichtigen Hirnregionen, wie dem Hippocampus und dem cerebralen Cortex (Séguéla et al., *J. Neurosci.* **1993,** *13*, 596-604). Der α7-nAChR besitzt eine besonders hohe Durchlässigkeit für Calcium-Ionen, erhöht glutamaterge Neurotransmission, beeinflusst das Wachstum von Neuriten und moduliert auf diese Weise die neuronale Plastizität (Broide und Leslie, *Mol. Neurobiol*. **1999**, *20*, 1-16).

Bestimmte Chinuclidincarbonsäureanilide sind als Antiarrhythmika und Lokalanästhetika beschrieben (vgl. beispielsweise FR 1.566.045, GB 1 578 421 und Oppenheimer et al., *Life Sci*. **1991**, *48*, 977-985).

Die WO 01/60821 offenbart Biarylcarbonsäureamide mit Affinität zum α7-nAChR zur Behandlung von Lern- und Wahrnehmungsstörungen.

Weitere Verbindungen mit gleicher aktivität werden in US 5 998 429 offenbart.

Die vorliegende Erfindung betrifft Verbindungen der allgemeinen Formel (I) 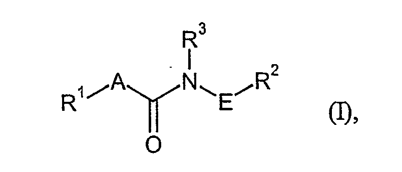 in welcher
- R¹: für einen 1-Aza-bicyclo[m.n.p]alkyl-Rest mit 7 bis 11 Ringatomen steht,
worin m und n unabhängig voneinander 2 oder 3 bedeuten,
worin p 1, 2 oder 3 bedeutet,
und wobei der Bicycloalkylrest gegebenenfalls durch (C₁-C₆)-Alkyl substituiert ist,
- A: für eine Bindung, Methylen, Ethylen oder Propylen steht,
- E: für zweibindiges, 5- bis 6-gliedriges Heteroaryl oder Benzoldiyl steht, wobei Heteroaryl und Benzoldiyl gegebenenfalls durch Reste ausgewählt aus der Gruppe Halogen, Cyano, Trifluormethyl, Trifluormethoxy und (C₁-C₆)-Alkyl substituiert sind,
- R²: für 5- bis 6-gliedriges Heteroaryl oder Phenyl steht, wobei Heteroaryl und Phenyl gegebenenfalls durch Reste ausgewählt aus der Gruppe Halogen, 5-bis 6-gliedriges Heterocyclyl, -CO-NR⁴R⁵, -CO-OR⁶, -NR⁷R⁸, -NR⁹-CO-R¹⁰, -COR¹³, Cyano, Trifluormethyl, Trifluormethoxy, Nitro, gegebenenfalls durch Hydroxyl, Amino, NH-CO-R¹¹, -O-CO-NHR¹⁴, Halogen oder Cyano substituiertes (C₁-C₆)-Alkyl, (C₁-C₆)-Alkoxy und (C₁-C₆)-Alkylthio substituiert sind,
worin R⁴, R⁵, R⁶, R⁷, R⁸, R⁹, R¹⁰ R¹¹, R¹³ und R¹⁴ unabhängig voneinander Wasserstoff, (C₁-C₆)-Alkyl, Phenyl oder Benzyl bedeuten,
und
- R³: für Wasserstoff oder (C₁-C₆)-Alkyl steht,
und deren Salze, Solvate und Solvate der Salze.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Die erfindungsgemäßen Verbindungen können auch in Form ihrer Salze, Solvate oder Solvate der Salze vorliegen.

Als Salze sind im Rahmen der Erfindung physiologisch unbedenkliche Salze der erfindungsgemäßen Verbindungen bevorzugt.

Physiologisch unbedenkliche Salze der erfindungsgemäßen Verbindungen können Säureadditionssalze der Verbindungen mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können aber auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder N-Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten im Allgemeinen die folgende Bedeutung:
(C₁-C₆)- und (C₁-C₄)-Alkoxy steht für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4, besonders bevorzugt mit 1 bis 3 Kohlenstoffatomen. Beispielsweise und vorzugsweise seien genannt: Methoxy, Ethoxy, n-Propoxy, Isopropoxy, tert.-Butoxy, n-Pentoxy und n-Hexoxy.
(C₁-C₆)- und (C₁-C₄)-Alkyl stehen für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4, besonders bevorzugt mit 1 bis 3 Kohlenstoffatomen.

Beispielsweise und vorzugsweise seien genannt: Methyl, Ethyl, n-Propyl, Isopropyl, tert.-Butyl, n-Pentyl und n-Hexyl.

(C₁-C₆)-Alkylthio steht für einen geradkettigen oder verzweigten Alkylthiorest mit 1 bis 6 Kohlenstoffatomen. Bevorzugt ist ein geradkettiger oder verzweigter Alkylthiorest mit 1 bis 4, besonders bevorzugt mit 1 bis 3 Kohlenstoffatomen. Beispielsweise und vorzugsweise seien genannt: Methylthio, Ethylthio, n-Propylthio, Isopropylthio, tert.-Butylthio, n-Pentylthio und n-Hexylthio.

Der 1-Aza-bicyclo[m.n.p]alkyl-Rest mit 7 bis 11 Ringatomen ist vorzugsweise und beispielhaft: 1-Aza-bicyclo[3.2.1]octyl (Isotropan), 1-Aza-bicyclo[3.3.1]nonyl (Isogranatan), 1-Aza-bicyclo[2.2.2]octyl (Chinuclidin).

Halogen steht für Fluor, Chlor, Brom und Jod. Bevorzugt sind Fluor, Chlor und Brom. Besonders bevorzugt sind Fluor und Chlor.

5- bis 6-gliedriges Heteroaryl steht für einen aromatischen Rest mit 5 bis 6 Ringatomen und bis zu 4, vorzugsweise bis zu 2 Heteroatomen aus der Reihe S, O und/oder N. Der Heteroarylrest kann über ein Kohlenstoff- oder Heteroatom gebunden sein. Beispielsweise und vorzugsweise seien genannt: Thienyl, Furyl, Pyrrolyl, Thiazolyl, Oxazolyl, Imidazolyl, Pyridyl, Pyrimidinyl, und Pyridazinyl.

Zweibindiges 5- bis 6-gliedriges Heteroaryl steht für einen zweibindigen aromatischen Rest mit 5 bis 6 Ringatomen und bis zu 4, vorzugsweise bis zu 2 Heteroatomen aus der Reihe S, O und/oder N. Der Heteroarylrest kann über ein Kohlenstoff-oder Heteroatom gebunden sein. Beispielsweise und vorzugsweise seien genannt: Thiophendiyl, Furandiyl, Pyrroldiyl, Thiazoldiyl, Oxazoldiyl, Imidazoldiyl, Pyridindiyl, Pyrimidindiyl, und Pyridazindiyl.

5- bis 6-gliedriges Heterocyclyl steht für einen heterocyclischen Rest mit 5 bis 6 Ringatomen und bis zu 3, vorzugsweise 2 Heteroatomen bzw. Heterogruppen aus der Reihe N, O, S, SO, SO₂, bevorzugt sind N und O. Die Heterocyclyl-Reste können gesättigt oder teilweise ungesättigt sein. Gesättigte Heterocyclyl-Reste sind bevorzugt. Die Heterocyclyl-Reste können über ein Kohlenstoffatom oder ein Heteroatom gebunden sein. Nicht-limitierende Beispiele umfassen Pyrrolinyl, Tetrahydrofuranyl, Tetrahydrothienyl, Pyranyl, Piperidinyl, Piperazinyl, Thiopyranyl, Morpholinyl.

Wenn Reste in den erfindungsgemäßen Verbindungen gegebenenfalls substituiert sind, können die Reste, soweit nicht anders spezifiziert, ein- oder mehrfach gleich oder verschieden substituiert sein. Eine Substitution mit bis zu drei gleichen oder verschiedenen Substituenten ist bevorzugt.

Bevorzugt sind Verbindungen der Formel (I),
in welcher
- R¹: für 1-Aza-bicyclo[2.2.2]oct-3-yl steht,
- A: für eine Bindung oder Methylen steht,
- E: für Benzoldiyl, das gegebenenfalls durch einen Rest ausgewählt aus der Gruppe Fluor, Chlor, Cyano, Methyl und Trifluormethyl substituiert ist, steht,
- R²: für Thienyl oder Phenyl steht, wobei die Ringe gegebenenfalls mit bis zu 2 Resten ausgewählt aus der Gruppe Halogen, Cyano, Trifluormethyl, Trifluormethoxy, Nitro, Morpholinyl, (C₁-C₄)-Alkyl, (C₁-C₄)-Alkoxy, (C₁-C₄)-Alkylthio, -CO-NR⁴R⁵, -CO-OR⁶, -NR⁹-CO-R¹⁰ und -CO-R¹³ substituiert sind,
wobei (C₁-C₄)-Alkyl gegebenenfalls mit Hydroxy, Halogen und -O-CO-NHR¹⁴ substituiert ist,
wobei R⁴, R⁵, R⁶, R⁹, R¹⁰, R¹³ und R¹⁴ unabhängig voneinander für
Wasserstoff oder (C₁-C₄)-Alkyl stehen,
und
- R³: für Wasserstoff steht, sowie deren Salze, Solvate und Solvate der Salze.

Bevorzugt sind Verbindungen der allgemeinen Formel (I), in welcher
- R¹: für 1-Aza-bicyclo[2.2.2]octyl steht.

Besonders bevorzugt sind Verbindungen der allgemeinen Formel (I), in welcher
- R¹: für 1-Aza-bicyclo[2.2.2]oct-3-yl steht.

Ebenfalls bevorzugt sind Verbindungen der allgemeinen Formel (I), in welcher
- A: für eine Bindung oder Methylen steht.

Besonders bevorzugt sind Verbindungen der allgemeinen Formel (I), in welcher
- A: für eine Bindung steht.

Ebenfalls bevorzugt sind Verbindungen der allgemeinen Formel (I), in welcher
- E: für Benzoldiyl, das gegebenenfalls durch 1 bis 3 Reste ausgewählt aus der Gruppe Fluor, Chlor, Cyano, Methyl und Trifluormethyl substituiert ist, steht.

Besonders bevorzugt sind Verbindungen der allgemeinen Formel (I), in welcher
- E: für Benzoldiyl steht.

Ebenfalls bevorzugt sind Verbindungen der allgemeinen Formel (I), in welcher
- R²: für Thienyl oder Phenyl steht, wobei die Ringe gegebenenfalls durch 1 bis 3 Reste ausgewählt aus der Gruppe Halogen, Cyano, Trifluormethyl, Trifluormethoxy, Nitro, (C₁-C₄)-Alkyl, Hydroxymethyl, und (C₁-C₄)-Alkoxy substituiert sind.

Besonders bevorzugt sind Verbindungen der allgemeinen Formel (I), in welcher
- R²: für Hydroxymethylphenyl steht.

Ebenfalls bevorzugt sind Verbindungen der allgemeinen Formel (I), in welcher
- R³: für Wasserstoff oder Methyl steht.

Besonders bevorzugt sind Verbindungen der allgemeinen Formel (I), in welcher
- R³: für Wasserstoff steht.

Ganz besonders bevorzugt sind Kombinationen von zwei oder mehreren der oben genannten Vorzugsbereiche.

Ebenfalls ganz besonders bevorzugt sind Verbindungen der allgemeinen Formel (I), in welcher
- R¹: für 1-Aza-bicyclo[2.2.2]oct-3-yl steht,
- A: für eine Bindung steht,
- E: für Benzoldiyl, das gegebenenfalls durch 1 bis 3 Reste ausgewählt aus der Gruppe Fluor, Chlor, Cyano, Methyl und Trifluormethyl substituiert ist, steht,
- R²: für Thienyl oder Phenyl steht, wobei die Ringe gegebenenfalls durch 1 bis 3 Reste ausgewählt aus der Gruppe Halogen, Cyano, Trifluormethyl, Trifluormethoxy, Nitro, (C₁-C₄)-Alkyl, Hydroxymethyl, und (C₁-C₄)-Alkoxy substituiert sind,
und
- R³: für Wasserstoff steht.

Die Erfindung betrifft weiterhin Verfahren zur Herstellung der Verbindungen der Formel (I), dadurch gekennzeichnet, dass man
[A] Verbindungen der allgemeinen Formel (II) 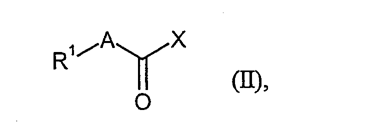 in welcher
   - R¹ und A: die oben angegebenen Bedeutungen haben, und
   - X: für Hydroxy oder eine geeignete Abgangsgruppe wie beispielsweise Chlor oder Pentaflourphenoxy steht,
   mit einer Verbindung der allgemeinen Formel (III)

   R³-NH-E-R² (III),

   in welcher
   - R², R³ und E: die oben angegebenen Bedeutungen haben,
   in einem inerten Lösungsmittel gegebenenfalls in Gegenwart eines Kondensationsmittels und gegebenenfalls in Gegenwart einer Base umsetzt, oder
[B] Verbindungen der allgemeinen Formel (II) zunächst mit einer Verbindung der allgemeinen Formel (IV)

   R³-NH-E-Y (IV),
in welcher
- R³ und E: die oben angegebenen Bedeutungen haben, und
- Y: für eine geeignete Abgangsgruppe wie beispielsweise Triflat oder Halogen, vorzugsweise Brom oder Iod, steht,
gegebenenfalls in einem inerten Lösungsmittel gegebenenfalls in Gegenwart eines Kondensationsmittels und gegebenenfalls in Gegenwart einer Base zu Verbindungen der allgemeinen Formel (V) 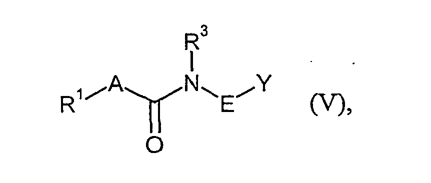 in welcher
- R¹, R³, A, E und Y: die oben angegebenen Bedeutungen haben,
umsetzt,
und diese dann in einer Kupplungsreaktion mit Verbindungen der allgemeinen Formel (VI) 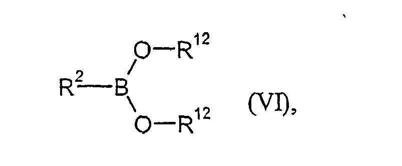 in welcher
- R²: die oben angegebenen Bedeutungen hat, und
- R¹²: für Wasserstoff oder Methyl steht oder beide Reste zusammen eine CH₂CH₂- oder C(CH₃)₂-C(CH₃)₂-Brücke bilden,
in einem inerten Lösungsmittel in Gegenwart eines geeigneten Katalysators und in Gegenwart einer Base umsetzt und die resultierenden Verbindungen der Formel (I) gegebenenfalls mit den entsprechenden (i) Lösungsmitteln und/oder (ii) Basen oder Säuren zu ihren Solvaten, Salzen und/oder Solvaten der Salze umsetzt.

Wenn X eine Abgangsgruppe ist, sind Chloro, Mesyloxy und Isobutyloxycarbonyloxy, besonders Chloro bevorzugt.

Inerte Lösungsmittel für die Verfahrensschritte (II) + (III) → (I) und (II) + (IV) → (V) sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Nitromethan, Ethylacetat, Aceton, Dimethylformamid, Dimethylacetamid, Dimethylsulfoxid, Acetonitril oder Pyridin. Bevorzugt ist Dimethylformamid, Tetrahydrofuran, Methylenchlorid oder Chloroform.

Kondensationsmittel für die Verfahrensschritte (II) + (III) → (I) und (II) + (IV) → (V) sind beispielsweise Carbodiimide wie z.B. N,N'-Diethyl-, N,N'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformiat, oder Bis-(2-oxo-3-oxazolidinyl)-phosphorylchlorid oder Benzotriazolyloxy-tri(dimethylamino)phosphonium-hexafluorophosphat, oder O-(Benzotriazol-1-yl)-N,N,N',N'-tetramethyluronium-hexafluorophosphat (HBTU), 2-(2-Oxo-1-(2H)-pyridyl)-1,1,3,3-tetramethyluroniumtetrafluoroborat (TPTU) oder O-(7-Azabenzotriazol-1-yl)-N,N,N',N'-tetramethyluronium-hexafluorophosphat (HATU) oder Benzotriazol-1-yloxytris(dimethylamino)-phosphonium-hexafluorophosphat (BOP), oder Mischungen aus diesen.

Gegebenenfalls kann es vorteilhaft sein, diese Kondensationsmittel in Gegenwart eines Hilfsnucleophils wie z.B. 1-Hydroxybenzotriazol (HOBt) zu verwenden.

Besonders bevorzugt ist HATU oder die Kombination von N-(3-Dimethylaminoisopropyl)-N'-ethylcarbodiimid-Hydrochlorid (EDC) und 1-Hydroxybenzotriazol (HOBt) in Dimethylformamid.

Basen für die Verfahrensschritte (II) + (III) → (I) und (II) + (IV) → (V) sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin.

Vorzugsweise werden die Verfahrensschritte (II) + (III) → (I) und (II) + (IV) → (V) in einem Temperaturbereich von Raumtemperatur bis 50°C bei Normaldruck durchgeführt.

Inerte Lösungsmittel für den Verfahrensschritt (V) + (VI) → (I) sind beispielsweise Ether wie Dioxan, Tetrahydrofuran oder 1,2-Dimethoxyethan, Kohlenwasserstoffe wie Benzol, Xylol oder Toluol, oder andere Lösemittel wie Nitrobenzol, Dimethylformamid, Dimethylacetamid, Dimethylsulfoxid oder N-Methylpyrrolidon. Bevorzugt sind Lösungsmittel wie z.B. Dimethylformamid, Dimethylacetamid, Dimethylsulfoxid oder 1,2-Dimethoxyethan.

Für den Verfahrensschritt (V) + (VI) → (I) geeignete Katalysatoren sind beispielsweise für Suzuki-Kupplungen übliche Palladium-Katalysatoren, bevorzugt sind Katalysatoren wie z.B. Dichlorbis(triphenylphosphin)palladium, Tetrakistriphenylphosphinpalladium, Palladium(II)acetat oder Bis-(diphenylphosphino)ferrocen-palladium(II)chlorid (vgl. z.B. A. Suzuki, *Acc. Chem. Res.* **1982,** *15*, 178ff; Miyaura et al., *J. Am. Chem. Soc*. **1989,** *111*, 314).

Für den Verfahrensschritt (V) + (VI) → (I) geeignete Basen sind beispielsweise Kaliumacetat, Cäsium-, Kalium- oder Natriumcarbonat, Bariumhydroxid, Kalium-tert.-butylat, Cäsiumfluorid oder Kaliumphosphat. Bevorzugt ist Cäsiumcarbonat oder Natriumcarbonat.

Vorzugsweise wird der Verfahrensschritt (V) + (VI) → (I) in einem Temperaturbereich von Raumtemperatur bis 130°C bei Normaldruck durchgeführt.

Die Verbindungen der allgemeinen Formeln (II) und (VI) sind bekannt oder lassen sich nach bekannten Verfahren aus den entsprechenden Edukten synthetisieren [vgl. z.B. für Verbindungen der allgemeinen Formel (II): Kato et al., *Chem. Pharm. Bull.* **1995,** *43,* 1351-1357; Orlek et al., *J. Med. Chem*. **1991,** *34,* 2726-2735; Plate et al., *Bioorg. Med. Chem*. **2000,** *8,* 449-454; für Verbindungen der allgemeinen Formel (VI): D.S. Matteson, in: *Stereodirected Synthesis with Organoboranes*, Hrsg. K. Hafner, C.W. Rees, B.M. Trost, J.-M. Lehn, P. v. Ragué Schleyer, Springer-Verlag, Heidelberg 1995; H.C. Brown, G.W. Kramer, A.B. Levy, M.M. Midland, *Organic Synthesis via Boranes*, Wiley, New York 1975; A. Pelter, K. Smith, H.C. Brown, *Borane Reagents,* Academic Press, London 1988].

Die Verbindungen der allgemeinen Formeln (III) und (IV) sind ebenfalls bekannt oder lassen sich nach bekannten Verfahren aus den entsprechenden Edukten synthetisieren (vgl. z.B. *Comprehensive Heterocyclic Chemistry*, Katritzky et al., Hrsg., Elsevier, 1996). So können beispielsweise Benzoesäurederivate gemäß folgendem Syntheseschema via Umlagerung (Curtius-Abbau) der korrespondierenden Carbonsäureazide in die entsprechenden Anilinderivate überführt werden (vgl. z.B. S. Deprets, G. Kirsch, *Eur. J. Org. Chem.* **2000**, *7,* 1353ff.):

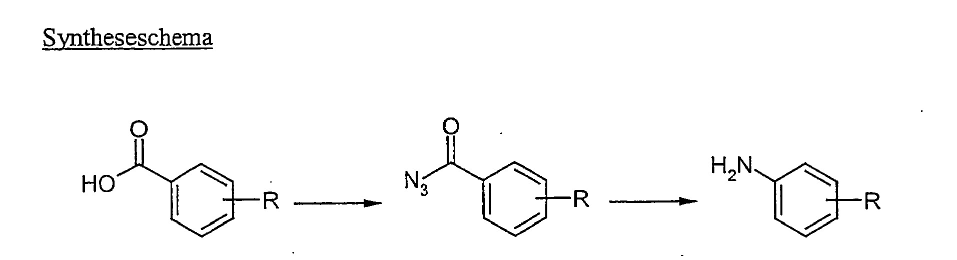

Die erfindungsgemäßen Verbindungen der allgemeinen Formel (I) eignen sich zur Verwendung als Arzneimittel zur Behandlung und/oder Prophylaxe von Krankheiten bei Menschen und/oder Tieren.

Die erfindungsgemäßen Verbindungen zeigen ein nicht vorhersehbares, wertvolles pharmakologisches Wirkspekrtrum.

Sie zeichnen sich als Liganden, insbesondere als Agonisten am α7-nAChR aus.

Die erfindungsgemäßen Verbindungen können aufgrund ihrer pharmakologischen Eigenschaften allein oder in Kombination mit anderen Arzneimitteln zur Behandlung und/oder Prävention von kognitiven Störungen, insbesondere der Alzheimer'schen Krankheit eingesetzt werden. Wegen ihrer selektiven Wirkung als α7-nAChR-Agonisten eignen sich die erfindungsgemäßen Verbindungen besonders zur Verbesserung der Wahrnehmung, Konzentrationsleistung, Lernleistung, oder Gedächtnisleistung insbesondere nach kognitiven Störungen, wie sie beispielsweise bei Situationen/Krankheiten/Syndromen auftreten wie "Mild cognitive impairment", Altersassoziierte Lern- und Gedächtnisstörungen, Altersassoziierte Gedächtnisverluste, Vaskuläre Demenz, Schädel-Hirn-Trauma, Schlaganfall, Demenz, die nach Schlaganfällen auftritt ("post stroke dementia"), post-traumatisches Schädel-Hirn-Trauma, allgemeine Konzentrationsstörungen, Konzentrationsstörungen bei Kindern mit Lern- und Gedächtnisproblemen, Attention Deficit Hyperactivity Disorder, Alzheimer'sche Krankheit, Demenz mit Lewy-Körperchen, Demenz mit Degeneration der Frontallappen einschließlich des Pick's Syndroms, Parkinson'sche Krankheit, Progressive nuclear palsy, Demenz mit corticobasaler Degeneration, Amyotrophe Lateralsklerose (ALS), Huntington'sche Krankheit, Multiple Sklerose, Thalamische Degeneration, Creutzfeld-Jacob-Demenz, HIV-Demenz, Schizophrenie, Schizophrenie mit Demenz oder Korsakoff-Psychöse.

Die erfindungsgemäßen Verbindungen können allein oder in Kombination mit anderen Wirkstoffen zur Prävention und Behandlung der Folgen von neurodegenerativen Erkrankungen eingesetzt werden. Als nicht-limitierende Beispiele für neurodegenerative Erkrankungen seien Alzheimer'sche Krankheit und Parkinson'sche Krankheit genannt.

Die erfindungsgemäßen Verbindungen können allein oder in Kombination mit anderen Arzneimitteln eingesetzt werden zur Prophylaxe und Behandlung von akuten und/oder chronischen Schmerzen (für eine Klassifizierung siehe "Classification of Chronic Pain, Descriptions of Chronic Pain Syndromes and Definitions of Pain Terms", 2. Aufl., Meskey und Begduk, Hrsg.; IASP-Press, Seattle, 1994), insbesondere zur Behandlung von Krebs-induzierten Schmerzen und chronischen neuropathischen Schmerzen, wie zum Beispiel bei diabetischer Neuropathie, postherpetischer Neuralgie, peripheren Nervenbeschädigungen, zentralem Schmerz (beispielsweise als Folge von cerebraler Ischämie) und trigeminaler Neuralgie, und anderen chronischen Schmerzen, wie zum Beispiel Lumbago, Rückenschmerz (low back pain) oder rheumatischen Schmerzen. Daneben eignen sich diese Substanzen auch zur Therapie von primär akuten Schmerzen jeglicher Genese und von daraus resultierenden sekundären Schmerzzuständen, sowie zur Therapie chronifizierter, ehemals akuter Schmerzzustände.

Die *in vitro*-Wirkung der erfindungsgemäßen Verbindungen kann in folgenden Assays gezeigt werden:

### 1. Bestimmung der Affinität von Testsubstanzen für α7-nAChR durch Inhibition von [³H]Methyllycaconitine-Bindung an Rattenhirnmembranen

Der [³H]-Methyllycaconitine Bindungstest ist eine Modifikation der von Davies et al. (*Neuropharmacol*. **1999**, *38*, 679-690) beschriebenen Methode.

Rattenhirngewebe (Hippocampus oder Gesamthirn) wird in Homogenisierungspuffer (10% w/v) [0.32 M Sucrose, 1 mM EDTA, 0.1 mM Phenylmethylsulfonylfluorid (PMSF), 0.01 % (w/v) NaN₃, pH 7.4, 4°C] bei 600 rpm in einem Glashomogenisator homogenisiert. Das Homogenat wird zentrifugiert (1000 x g, 4°C, 10 min) und der Überstand wird abgenommen. Das Pellet wird erneut suspendiert (20% w/v) und zentrifugiert (1000 x g, 4°C, 10 min). Die beiden Überstände werden vereinigt und zentrifugiert (15.000 x g, 4°C, 30 min). Dieses Pellet wird als P2-Fraktion bezeichnet.

Das P2-Pellet wird zweimal mit Bindungspuffer gewaschen (50 mM Tris-HCl, 1 mM MgCl₂, 120 mM NaCl, 5 mM KCl, 2 mM CaCl₂, pH 7.4) und zentrifugiert (15.000 x g, 4°C, 30 min).

Die P2-Membranen werden in Bindungspuffer resuspendiert und in einem Volumen von 250 µl (Membranproteinmenge 0.1-0.5 mg) für 2.5 h bei 21°C inkubiert in der Gegenwart von 1-5 nM [³H]-Methyllycaconitine, 0.1% (w/v) BSA (bovines Serumalbumin) und verschiedenen Konzentrationen der Testsubstanz. Die unspezifische Bindung wird bestimmt durch Inkubation in der Gegenwart von 1 µM α-Bungaro-toxin oder 100 µM Nicotin oder 10 µM MLA (Methyllycaconitine).

Die Inkubation wird beendet durch Zugabe von 4 ml PBS (20 mM Na₂HPO₄, 5 mM KH₂PO₄, 150 mM NaCl, pH 7.4, 4°C) und Filtration durch Typ A/E glass fibre filters (Gelman Sciences), die vorher 3 h in 0.3% (v/v) Polyethylenimin (PEI) eingelegt waren. Die Filter werden zweimal mit 4 ml PBS (4°C) gewaschen und die gebundene Radioaktivität durch Szintillationsmessung bestimmt. Alle Tests werden in Dreifachbestimmungen durchgeführt. Aus dem IC₅₀-Wert der Verbindungen (Konzentration der Testsubstanz, bei der 50% des am Rezeptor gebundenen Liganden verdrängt werden), der Dissoziationskonstante K_{D} und der Konzentration L von [³H]-Methyllycaconitine wird die Dissoziationskonstante der Testsubstanz Kᵢ bestimmt (Kᵢ = IC₅₀/ (1+L/K_{D})).

Anstelle von [³H]-Methyllycaconitine können auch andere α7-nAChR-selektive Radioliganden wie z.B. [¹²⁵I]-α-Bungarotoxin oder unselektive nAChR-Radioliganden gemeinsam mit Inhibitoren anderer nAChR eingesetzt werden.

Repräsentative in-vitro-Wirkdaten für die erfindungsgemäßen Verbindungen sind in Tabelle A wiedergegeben:

**Tabelle A**

| **Beispiel-Nr.** | **Kᵢ-Wert [nM]** |
|---|---|
| 9 | 519 |
| 14 | 20 |
| 15 | 39 |
| 20 | 10 |
| 21 | 98 |
| 22 | 58 |
| 23 | 39 |
| 24 | 230 |
| 26 | 310 |
| 31 | 420 |

Die Eignung der erfindungsgemäßen Verbindungen zur Behandlung von kognitiven Störungen kann in folgenden Tiermodellen gezeigt werden:

### 2. Objekt-Wiedererkennungstest

Der Objekt-Wiedererkennungstest ist ein Gedächtnistest. Er misst die Fähigkeit von Ratten (und Mäusen), zwischen bekannten und unbekannten Objekten zu unterscheiden.

Der Test wird wie beschrieben durchgeführt (Blokland et al., *NeuroReport* **1998,** *9,* 4205-4208; Ennaceur, A., Delacour, J., *Behav. Brain Res.* **1988,** *31,* 47-59; Ennaceur, A., Meliani, K., *Psychopharmacology* **1992,** *109,* 321-330; Prickaerts et al., *Eur. J. Pharmacol*. **1997,** *337*, 125-136).

In einem ersten Durchgang wird eine Ratte in einer ansonsten leeren größeren Beobachtungsarena mit zwei identischen Objekten konfrontiert. Die Ratte wird beide Objekte ausgiebig untersuchen, d.h. beschnüffeln und berühren. In einem zweiten Durchgang, nach einer Wartezeit von 24 Stunden, wird die Ratte erneut in die Beobachtungsarena gesetzt. Nun ist eines der bekannten Objekte durch ein neues, unbekanntes Objekt ersetzt. Wenn eine Ratte das bekannte Objekt wiedererkennt, wird sie vor allem das unbekannte Objekt untersuchen. Nach 24 Stunden hat eine Ratte jedoch normalerweise vergessen, welches Objekt sie bereits im ersten Durchgang untersucht hat, und wird daher beide Objekte gleichstark inspektieren. Die Gabe einer Substanz mit lern- und gedächtnisverbessernder Wirkung wird dazu führen, dass eine Ratte das bereits 24 Stunden vorher, im ersten Durchgang, gesehene Objekt als bekannt wiedererkennt. Sie wird das neue, unbekannte Objekt ausführlicher untersuchen als das bereits bekannte. Diese Gedächtnisleistung wird in einem Diskriminationsindex ausgedrückt. Ein Diskriminationsindex von Null bedeutet, dass die Ratte beide Objekte, das alte und das neue, gleichlang untersucht; d.h. sie hat das alte Objekt nicht wiedererkannt und reagiert auf beide Objekte als wären sie unbekannt und neu. Ein Diskriminationsindex größer Null bedeutet, dass die Ratte das neue Objekt länger inspektiert als das alte; d.h. die Ratte hat das alte Objekt wiedererkannt.

### 3. Sozialer Wiedererkennungstest:

Der Soziale Wiedererkennungstest ist ein Test zur Prüfung der lern- oder gedächtnisverbessernden Wirkung von Testsubstanzen.

Erwachsene Ratten, die in Gruppen gehalten werden, werden 30 Minuten vor Testbeginn einzeln in Testkäfige gesetzt. Vier Minuten vor Testbeginn wird das Testtier in eine Beobachtungsbox gebracht. Nach dieser Adaptationszeit wird ein juveniles Tier zu dem Testtier gesetzt und 2 Minuten lang die totale Zeit gemessen, die das adulte Tier das Junge investigiert (Trial 1). Gemessen werden alle deutlich auf das Jungtier gerichteten Verhaltensweisen, d.h. ano-genitale Inspektion, Verfolgen sowie Fellpflege, bei denen das Alttier einen Abstand von höchstens 1 cm zu dem Jungtier hat. Danach wird das Juvenile herausgenommen und das Adulte in seinem Testkäfig belassen (bei 24 Stunden Retention wird das Tier in seinen Heimkäfig zurückgesetzt). Vor oder nach dem ersten Test wird das Versuchstier mit Substanz behandelt. Je nach Zeitpunkt der Substanzgabe wird das Erlernen oder das Speichern der Information über das Jungtier durch die Substanz beeinflusst. Nach einem festgelegten Zeitraum (Retention) wird der Test wiederholt (Trial 2). Je größer die Differenz zwischen den in Trial 1 und 2 ermittelten Investigationszeiten, desto besser hat sich das adulte Tier an das Jungtier erinnert

Die erfindungsgemäßen Verbindungen der allgemeinen Formel (I) eignen sich zur Verwendung als Arzneimittel für Menschen und Tiere.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Verbindungen der Formel (I) bestehen, sowie Verfahren zur Herstellung dieser Zubereitungen.

Die Verbindungen der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Verbindungen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, insbesondere perlingual oder intravenös. Sie kann aber auch durch Inhalation über Mund oder Nase, beispielsweise mit Hilfe eines Sprays erfolgen, oder topisch über die Haut.

Im Allgemeinen hat es sich als vorteilhaft erwiesen, Mengen von etwa 0,001 bis 10 mg/kg, bei oraler Anwendung vorzugsweise etwa 0,005 bis 3 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

### Abkürzungen:

- DCI: direkte chemische Ionisation (bei MS)
- DMF: *N*,*N*-Dimethylformamid
- DMSO: Dimethylsulfoxid
- d.Th.: der Theorie (bei Ausbeute)
- EDC: *N'*-(3-Dimethylaminopropyl)-*N*-ethylcarbodiimid x HCl
- eq.: Äquivalent(e)
- ESI: Elektrospray-Ionisation (bei MS)
- HPLC: Hochdruck-, Hochleistungsflüssigchromatographie
- LC-MS: Flüssigchromatographie-gekoppelte Massenspektroskopie
- MS: Massenspektroskopie
- NMR: Kernresonanzspektroskopie
- PdCl₂(dppf): 1,1'-Bis(diphenylphosphino)ferrocenpalladium(II)chlorid
- RT: Raumtemperatur
- Rₜ: Retentionszeit (bei HPLC)
- THF: Tetrahydrofuran

### LC-MS Methode A:

| | | | | |
|---|---|---|---|---|
| Gerätetyp MS: | Micromass Quattro LCZ | | | |
| | Ionisierung: ESI positiv | | | |
| Gerätetyp HPLC: | HP 1100 | | | |
| | UV-Detektor DAD: 208-400 nm | | | |
| | Ofentemp.: 40°C | | | |
| Säule: | Symmetry C 18 | | | |
| | 50 mm x 2.1 mm; 3.5 µm | | | |
| Gradient: | Zeit (min) | A: % | B: % | Fluss (mL/min) |
| | 0.00 | 10.0 | 90.0 | 0.50 |
| | 4.00 | 90.0 | 10.0 | 0.50 |
| | 6.00 | 90.0 | 10.0 | 0.50 |
| | 6.10 | 10.0 | 90.0 | 1.00 |
| | 7.50 | 10.0 | 90.0 | 0.50 |
| Eluent A: | Acetonitril + 0.1% Ameisensäure | | | |
| Eluent B: | Wasser + 0.1% Ameisensäure | | | |

### LC-MS Methode B:

| | | | | |
|---|---|---|---|---|
| Gerätetyp MS: | Micromass Platform LCZ | | | |
| | Ionisierung: ESI positiv | | | |
| Gerätetyp HPLC: | HP 1100 | | | |
| | UV-Detektor DAD: 208-400 nm | | | |
| | Ofentemp.: 40°C | | | |
| Säule: | Symmetry C 18 | | | |
| | 50 mm x 2.1 mm; 3.5 µm | | | |
| Gradient: | Zeit (min) | A: % | B: % | Fluss (mL/min) |
| | 0.00 | 10.0 | 90.0 | 0.50 |
| | 4.00 | 90.0 | 10.0 | 0.50 |
| | 6.00 | 90.0 | 10.0 | 0.50 |
| | 6.10 | 10.0 | 90.0 | 1.00 |
| | 7.50 | 10.0 | 90.0 | 0.50 |
| Eluent A: | Acetonitril + 0.1 % Ameisensäure | | | |
| Eluent B: | Wasser + 0.1% Ameisensäure | | | |

### LC-MS Methode C:

Instrument: Waters Alliance 2790 LC; Säule: Symmetry C18, 50 mm x 2.1 mm, 3.5 µm; Eluent A: Wasser + 0.1% Ameisensäure, Eluent B: Acetonitril + 0.1 % Ameisensäure; Gradient: 0.0 min 5% B → 5.0 min 10% B → 6.0 min 10% B; Temperatur: 50°C; Fluss: 1.0 mL/min; UV-Detektion: 210 nm.

### LC-MS Methode D:

Instrument: Micromass Platform LCZ mit HPLC Agilent Serie 1100; Säule: Grom-SIL120 ODS-4 HE, 50 mm x 2.0 mm, 3 µm; Eluent A: 1 L Wasser + 1 mL 50%-ige Ameisensäure, Eluent B: 1 L Acetonitril + 1 mL 50%-ige Ameisensäure; Gradient: 0.0 min 100% A → 0.2 min 100% A → 2.9 min 30% A → 3.1 min 10% A → 4.5 min 10% A; Ofen: 55°C; Fluss: 0.8 mL/min; UV-Detektion: 208-400 nm.

### HPLC-Methode E:

Instrument: HP 1100 mit DAD-Detektion; Säule: Kromasil RP-18, 60 mm x 2 mm, 3.5 µm; Eluent A: 5 mL HClO₄/L Wasser, Eluent B: Acetonitril; Gradient: 0 min 2% B → 0.5 min 2% B → 4.5 min 90% B → 6.5 min 90% B; Fluss: 0.75 mL/min; Temperatur: 30°C; UV-Detektion: 210 nm.

### LC-MS Methode F:

Instrument MS: Micromass TOF (LCT); Instrument HPLC: 2-Säulen-Schaltung, Waters 2690; Säule: YMC-ODS-AQ, 50 mm x 4.6 mm, 3.0 µm; Eluent A: Wasser + 0.1% Ameisensäure, Eluent B: Acetonitril + 0.1% Ameisensäure; Gradient: 0.0 min 100% A → 0.2 min 95% A → 1.8 min 25% A → 1.9 min 10% A → 3.2 min 10% A; Fluss: 3.0 mL/min; Ofen: 40°C; UV-Detektion: 210 nm.

### Ausgangsverbindungen:

### Beispiel 1A

### 3-Chinuklidincarbonsäurechlorid-Hydrochlorid

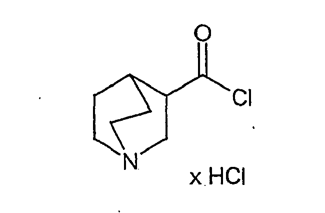

500 mg (2.61 mmol) 3-Chinuklidincarbonsäure-Hydrochlorid (Orlek et al., *J. Med. Chem.* **1991**, *34,* 2726) werden zusammen mit 1.9 mL (26.09 mmol) Thionylchorid 2 h unter Rückfluss gekocht. Das Reaktionsgemisch wird unter reduziertem Druck vom Thionylchlorid befreit. Es wird zweimal mit je 20 mL Toluol versetzt und bis zur Trockene eingeengt. Das so erhaltene Produkt wird ohne weitere Reinigung weiter umgesetzt.

### Beispiel 2A

### Chinuklidin-3-on

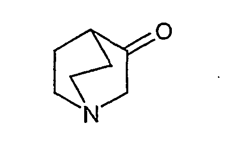

100 g (0.62 mol) Chinuklidin-3-on-Hydrochlorid werden in 2 L Methanol suspendiert. Bei 0°C wird eine Lösung von 33.4 g (0.62 mol) Natriummethylat in 250 mL Methanol langsam zugetropft. Es wird 16 h bei Raumtemperatur nachgerührt. Der entstandene Niederschlag wird abgesaugt, das Filtrat wird im Vakuum eingeengt. Der Rückstand wird zwischen Chloroform und Wasser verteilt und mit Chloroform extrahiert. Die vereinigten organischen Phasen werden über Natriumsulfat getrocknet und im Vakuum eingeengt. Man erhält 58.8 g (75.9% d.Th.) der Titelverbindung.
MS (DCI): *m*/*z* =126 (M+H)⁺, 143 (M+NH₄)⁺
¹H-NMR (300 MHz, CDCl₃): δ = 3.30 (m, 2H), 3.19-2.86 (m, 4H), 2.46 (m, 1H), 1.99 (m, 4H).

### Beispiel 3A

### Methyl (2Z)-1-azabicyclo[2.2.2]oct-3-yliden-ethanoat-Hydrochlorid

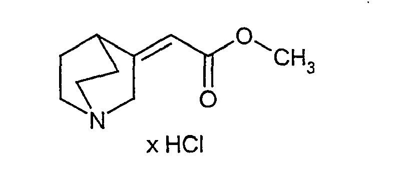

25.3 g (0.63 mol) Natriumhydrid (als 60%-ige Suspension in Mineralöl) werden in 480 mL Dimethylformamid suspendiert. Nach dem Hinzutropfen einer Lösung von 104.8 g (0.58 mol) Phosphonoessigsäuretrimethylester in 480 mL Dimethylformamid wird bis zur Beendigung der Wasserstoffentwicklung bei Raumtemperatur nachgerührt. Eine Lösung von 36 g (0.29 mol) Chinuklidin-3-on in 480 mL Dimethylformamid wird über einen Zeitraum von 40 Minuten hinzugetropft und anschließend für 16 h bei Raumtemperatur nachgerührt. Das Reaktionsgemisch wird im Vakuum eingeengt, der Rückstand zwischen Wasser und Essigsäureethylester verteilt und mit Essigsäureethylester extrahiert. Die vereinigten organischen Phasen werden über Natriumsulfat getrocknet und im Vakuum eingeengt. Der Rückstand wird an Kieselgel säulenchromatographisch gereinigt (Laufmittel: Dichlormethan/Methanol/ Ammoniak 95:5:0.5). Das erneut eingeengte Material wird in wenig Dichlormethan gelöst und mit etherischer HCl versetzt. Der entstehende Niederschlag wird abgesaugt und mit Diethylether nachgewaschen. Nach der Trocknung bei 35°C werden 19.53 g (31.2% d.Th.) der Titelverbindung in Form weißer Kristalle erhalten. HPLC (Kromasil RP-18, 60 x 2.1 mm; Eluent A: H₂O + 5 mL HClO₄/L, Eluent B: Acetonitril; Gradient: 0 - 4.5 min 98% A → 90% B, 4.5 - 6.5 min 90% B; Fluss: 0.75 mL/min; Temp.: 30°C; UV-Detektion bei 210 nm): Rₜ = 2.40 min.
MS (DCI): *m*/*z* = 182 (M+H)⁺, 199 (M+NH₄)⁺, 363 (2M+H)⁺
¹H-NMR (500 MHz, DMSO-d₆): δ = 11.56 (breites s, 1H), 5.97 (m, 1H), 4.32 (m, 2H), 3.66 (s, 3H), 3.27 (m, 4H), 2.84 (m, 1H), 2.13-1.92 (m, 2H), 1.91-1.69 (m, 2H); ¹³C-NMR (125 MHz, DMSO-d₆): δ = 165.72, 155.95, 113.08, 53.55, 51.28, 45.29, 30.14, 22.41.

### Beispiel 4A

### 1-Azabicyclo[2.2.2]oct-3-yl-essigsäure-Hydrochlorid

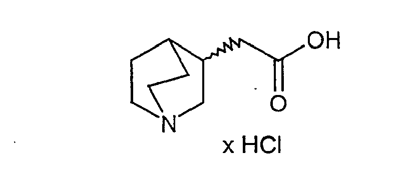

13.5 g (62 mmol) Methyl (2Z)-1-azabicyclo[2.2.2]oct-3-yliden-ethanoat werden in 200 mL Methanol gelöst und unter Argon mit 1 g Palladium auf Aktivkohle (10%-ig) versetzt. Bei Raumtemperatur wird das Reaktionsgemisch für 16 h unter Wasserstoffatmosphäre (Normaldruck) gerührt. Es wird über Kieselgur filtriert und mit Methanol nachgewaschen. Das Filtrat wird mit 50 mL 1 N Salzsäure versetzt, im Vakuum eingeengt und im Hochvakuum getrocknet. Der Rückstand wird in 100 mL 32%-iger Salzsäure für 5 h am Rückfluss erhitzt. Es wird im Vakuum eingeengt, zweimal mit Toluol codestilliert und im Hochvakuum getrocknet. Man erhält 11.8 g des Produkts in einer Reinheit von 89% (77% d.Th.).
HPLC (Kromasil RP-18, 60 x 2.1 mm; Eluent A: H₂O + 5 mL HClO₄/L, Eluent B: Acetonitril; Gradient: 0 - 4.5 min 98% A → 90% B, 4.5 - 6.5 min 90% B; Fluss: 0.75 mL/min; Temp.: 30°C; UV-Detektion bei 210 nm): Rₜ = 0.80 min.
MS (DCI): *m*/*z* = 170 (M+H)⁺, 339 (2M+H)⁺
¹H-NMR (200 MHz, DMSO-d₆): δ = 12.32 (breites s, 1H), 10.61 (breites s, 1H), 3.38 (m, 1H), 3.14 (m, 4H), 2.76 (dd, 1H), 2.67-2.22 (m, 4H), 2.01-1.55 (m, 4H).

### Beispiel 5A

### N-(4-Bromphenyl)-1-azabicyclo[2.2.2]octan-3-carboxamid-Hydrochlorid

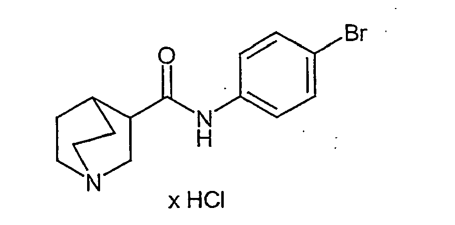

Zu einer Lösung aus 900 mg (5.24 mmol) 4-Bromanilin und 1.85 g (14.28 mmol) N,N-Diisopropylethylamin in etwa 10 mL trockenem DMF werden bei 0°C 1.00 g (4.76 mmol) 3-Chinuklidincarbonsäurechlorid-Hydrochlorid zugegeben. Es wird bei Raumtemperatur über Nacht nachgerührt. Nach Reinigung über präparative HPLC wird nochmals über Kieselgel chromatographiert (Laufmittel: Dichlormethan/Methanol/Triethylamin 80:20:2). Das Produkt wird in Methanol gelöst und mit 1 N HCl in Methanol versetzt. Schließlich wird das Solvens entfernt. Es werden 0.63 g (31 % d.Th.) der Titelverbindung erhalten. Das Rohmaterial wird direkt in die Folgesynthese eingesetzt.
LC-MS (Methode A): Rₜ = 2.33 min; MS (ESIpos): *m*/*z* = 309 (M+H)⁺ (freie Base).

### Beispiel 6A

### 2-(1-Azabicyclo[2.2.2]oct-3-yl)-N-(4-bromphenyl)acetamid-Hydrochlorid

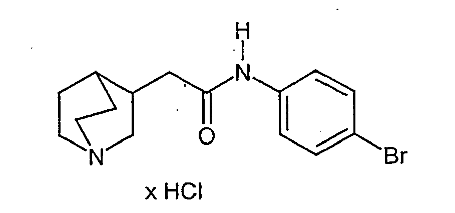

500 mg (2.43 mmol) 1-Azabicyclo[2.2.2]oct-3-yl-essigsäure werden in 10 mL Dichlormethan gelöst. Bei 0°C werden 1.79 g (9.72 mmol) Pentafluorphenol und 699 mg (3.65 mmol) N-[3-(Dimethylamino)propyl]-N'-ethylcarbodiimid-Hydrochlorid hinzugefügt. Nach 14 h Rühren bei Raumtemperatur wird im Vakuum eingeengt, der Rückstand in 8 mL Dimethylformamid gelöst, 627 mg (3.65 mmol) p-Bromanilin hinzugefügt und erneut 14 h bei Raumtemperatur gerührt. Es werden langsam 10 mL 10%-ige Natriumbicarbonat-Lösung und 10 mL Essigsäureethylester hinzugefügt. Das entstehende Präzipitat wird abgesaugt und das Filtrat dreimal mit Essigsäureethylester extrahiert. Nach dem Trocknen über Natriumsulfat entsteht beim Einengen ein Niederschlag, der abfiltriert wird. Der Feststoff wird in Dioxan suspendiert, mit 4 M HCl in Dioxan versetzt und 30 Minuten bei Raumtemperatur gerührt. Durch erneutes Absaugen des Feststoffs werden 621 mg (71% d.Th.) der Titelverbindung in Form weißer Kristalle erhalten.
HPLC (Kromasil RP-18, 60 x 2.1 mm; Eluent A: H₂O + 5 mL HClO₄/L, Eluent B: Acetonitril; Gradient = 0 - 4.5 min 98% A → 90% B, 4.5 - 6.5 min 90% B; Fluss: 0.75 mL/min; Temp.: 30°C; UV-Detektion bei 210 nm): Rₜ = 3.80 min.
MS (ESIpos): *m*/*z* = 323 (M+H)⁺ (freie Base)
¹H-NMR (200 MHz, DMSO-d₆): δ = 10.33 (s, 1H), 9.78 (breites s, 1H), 7.63-7.54 (m, 2H), 7.52-7.44 (m, 2H), 3.53-3.05 (m), 2.90 (dd, 1H), 2.71-2.35 (m), 2.00-1.60 (m, 5H).

### Beispiel 7A

### 2-(1-Azabicyclo[2.2.2]oct-3-yl)-N-(3-bromphenyl)acetamid-Hydrochlorid

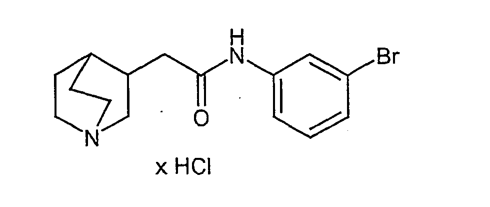

500 mg (2.34 mmol) 1-Azabicyclo[2.2.2]oct-3-ylessigsäure-Hydrochlorid werden in 10 mL Dichlormethan suspendiert und auf 0°C gekühlt. 1.79 g (9.72 mmol) Pentafluorphenol und 699.0 mg (3.65 mmol) EDC werden hinzugefügt, und es wird über Nacht bei Raumtemperatur gerührt. Nach Einengen im Vakuum versetzt man den Rückstand mit 8 mL DMF und 627.3 mg (3.56 mmol) 3-Bromanilin und lässt eine weitere Nacht bei Raumtemperatur rühren. Das Reaktionsgemisch wird mit 10 mL 10 %-iger wässriger Natriumbicarbonat-Lösung und 10 mL Essigsäureethylester verrührt. Nach Absaugen des entstandenen Niederschlags und Waschen mit Essigsäureethylester wird das zweiphasige Filtrat getrennt und die Wasserphase dreimal mit Essigsäureethylester extrahiert. Die vereinigten organischen Phasen werden über Natriumsulfat getrocknet und eingeengt. Das Rohgemisch wird mittels präparativer HPLC aufgereinigt. Die Produkt-Fraktionen werden eingeengt, in einem 5:1-Gemisch aus Methanol und 1 M Salzsäure aufgenommen und erneut eingeengt. Nach Trocknung im Hochvakuum erhält man 600 mg·(57.3% d.Th.) der Titelverbindung, welche ohne weitere Aufreinigung in die Folgestufen eingesetzt wird.
HPLC (Methode E): Rₜ = 3.8 min.
LC-MS (Methode C): *m*/*z* = 351 (M+H)⁺ (freie Base), Rₜ = 1.4 min.

### Beispiel 8A

### rac-1-Azabicyclo[2.2.2]octan-3-carbonitril

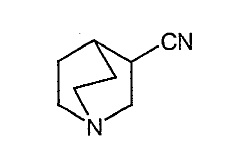

20.4 g (163 mmol) Chinuklidin-3-on und 41.37 g (211.87 mmol) (4-Toluolsulfonyl)-methylisocyanid werden in 435 mL 1,2-Dimethoxyethan und 16 mL trockenem Ethanol unter Eiskühlung vorgelegt. Es werden 45.72 g (407.45 mmol) Kalium-tert.-butylat langsam zugegeben, so dass die Temperatur auf maximal 10°C ansteigt. Anschließend wird 2.5 h lang auf 40°C erhitzt. Bei RT wird entstandener Feststoff abfiltriert. Das Filtrat wird eingeengt und über neutrales Aluminiumoxid chromatographiert (Laufmittel: zunächst Dichlormethan, dann Essigsäureethylester → Essigsäureethylester/Methanol 50:1). Es werden 22.9 g (103% d.Th.) des Produktes in leicht verunreinigter Form erhalten.

### Beispiel 9A

### S-1-Azabicyclo[2.2.2]octan-3-carbonitril

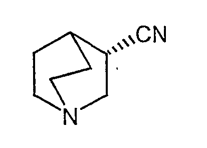

Die Trennung des Racemats aus Beispiel 8A in die Enantiomeren erfolgt mittels HPLC an chiraler Phase [Säule: Daicel Chiralpak AD 250 mm x 20 mm; Eluent: 5 % Wasser, 87 % Acetonitril, 8% Acetonitril mit 2% Diethylamin; Fluss: 10 mL/min; Injektionsvolumen: 0.3 mL; UV-Detektion: 220 nm]. Aus 20 g racemischen 1-Azabicyclo[2.2.2]octan-3-carbonitril werden 8.3 g (83 % d.Th.) der Titelverbindung erhalten.
HPLC (Säule: Chiralpak AD 250 mm x 4.6 mm, 10 µm; Eluent: 5% Wasser, 95 % Acetonitril mit 2 % Diethylamin; Temperatur: 30°C; Fluss: 1.0 mL/min): Rₜ = 5.24 min.

### Beispiel 10A

### R-1-Azabicyclo[2.2.2]octan-3-carbonitril

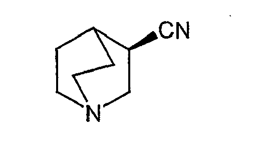

Aus 20 g racemischen 1-Azabicyclo[2.2.2]octan-3-carbonitril werden nach dem bei Beispiel 9A beschriebenen Verfahren 8.7 g (87% d.Th.) der Titelverbindung erhalten. HPLC (Säule: Chiralpak AD 250 mm x 4.6 mm, 10 µm; Eluent: 5% Wasser, 95% Acetonitril mit 2% Diethylamin; Temperatur: 30°C; Fluss: 1.0 mL/min): Rₜ = 6.19 min.

### Beispiel 11A

### S-1-Azabicyclo[2.2.2]octan-3-carbonsäure

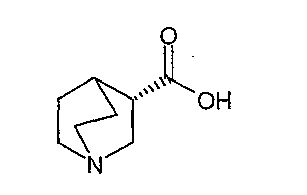

7.60 g (55.80 mmol) (*S*)-1-Azabicyclo[2.2.2]octan-3-carbonitril werden zusammen mit 80 mL konzentrierter Salzsäure 4 h lang unter Rückfluss erhitzt. Das Solvens wird unter reduziertem Druck entfernt und verbleibendes Restwasser mehrfach mit Toluol abdestilliert. Es werden 12.7 g Rohprodukt erhalten, das noch anorganische Salze enthält und ohne weitere Aufreinigung weiter umgesetzt wird.

### Beispiel 12A

### R-1-Azabicyclo[2.2.2]octan-3-carbonsäure

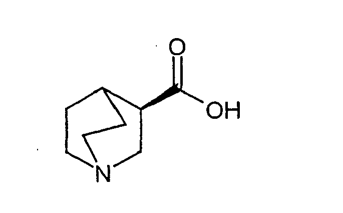

7.50 g (55.07 mmol) (*R*)-1-Azabicyclo[2.2.2]octan-3-carbonitril werden zusammen mit 78 mL konzentrierter Salzsäure 4 h lang unter Rückfluss erhitzt. Das Solvens wird unter reduziertem Druck entfernt und verbleibendes Restwasser mehrfach mit Toluol abdestilliert. Es werden 12.9 g Rohprodukt erhalten, das noch anorganische Salze enthält und ohne weitere Aufreinigung weiter umgesetzt wird.

### Beispiel 13A

### (3S)-N-(4-Bromphenyl)-1-azabicyclo[2.2.2]octan-3-carboxamid

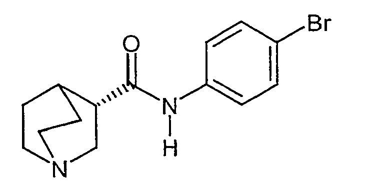

6.6 g (ca. 34.4 mmol) (*S*)-1-Azabicyclo[2.2.2]octan-3-carbonsäure werden zusammen mit 106 mL Thionylchlorid 1 h lang unter Rückfluss erhitzt. Das überschüssige Thionylchlorid wird unter reduziertem Druck entfernt und Reste azeotrop zusammen mit Toluol abdestilliert. Das so erhaltene rohe Säurechlorid wird zusammen mit 5.73 g (33.32 mmol) 4-Bromanilin und 23.21 mL (133.27 mmol) N,N-Diisopropylethylamin in 30 mL DMF 72 h lang bei RT gerührt. Das Solvens wird unter reduziertem Druck entfernt. Das Rohprodukt wird über Kieselgel 60 (Laufmittel: Dichlormethan → Dichlormethan/Methanol/Triethylamin 70:30:2) chromatographisch gereinigt. Das Solvens wird unter reduziertem Druck entfernt. Schließlich werden letzte Lösungsmittelreste im Hochvakuum entfernt. Es werden 2.9 g (28 % d.Th.) der Titelverbindung in einer Reinheit von 76% isoliert.
HPLC (Methode E): Rₜ = 3.84 min.

### Beispiel 14A

### (3R)-N-(4-Bromphenyl)-1-azabicyclo[2.2.2]octan-3-carboxamid

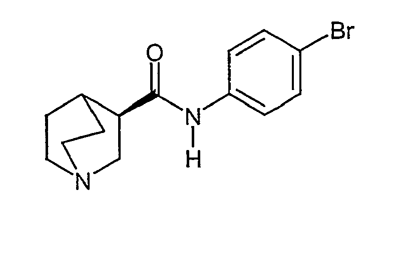

9.17 g (ca. 47.8 mmol) (*R*)-1-Azabicyclo[2.2.2]octan-3-carbonsäure werden zusammen mit 160 mL Thionylchlorid 1 h lang unter Rückfluss erhitzt. Das überschüssige Thionylchlorid wird unter reduziertem Druck entfernt und Reste azeotrop zusammen mit Toluol abdestilliert. Das so erhaltene rohe Säurechlorid wird zusammen mit 8.19 g (47.60 mmol) 4-Bromanilin und 24.6 mL (190.4 mmol) N,N-Diisopropylethylamin in 59 mL DMF 72 h lang bei RT gerührt. Das Solvens wird unter reduziertem Druck entfernt. Das Rohprodukt wird über Kieselgel 60 (Laufmittel: Dichlormethan → Dichlormethan/Methanol/Triethylamin 70:30:2) chromatographisch gereinigt. Das Solvens wird unter reduziertem Druck entfernt. Schließlich werden letzte Lösungsmittelreste im Hochvakuum entfernt. Es werden 5.5 g (37% d.Th.) der Titelverbindung erhalten. Die absolute Konfiguration wurde durch Kristallstrukturanalyse von Einkristallen zugeordnet.
¹H-NMR (200 MHz, DMSO-d₆): δ = 10.06 (s, 1H), 7.70-7.40 (m, 4H), 3.30-3.10 (m, 1H), 2.94-2.45 (m, 6H), 2.15-2.04 (m, 1H), 1.73-1.45 (m, 3H), 1.45-1.15 (m, 1H).
HPLC (Methode E): Rₜ = 3.84 min.
MS(ESIpos): *m*/*z* = 309 (M+H)⁺.

### Ausführungsbeispiele:

### Allgemeine Vorschrift zur Synthese der Ausführungsbeispiele 1-4:

1.0 eq. des Beispiels 5A, 1.2 eq. der entsprechenden Boronsäure, 0.1 eq. 1,1'-Bis(diphenylphosphino)ferrocenpalladium(II)chlorid und 2.2 eq. Caesiumcarbonat werden in 1,2-Dimethoxyethan 60 h auf 90°C erhitzt. Die Reinigung erfolgt chromatographisch durch präparative HPLC. Das aufgereinigte Produkt wird in Methanol gelöst und mit einem Überschuss an 1 *N* HCl in Methanol versetzt. Das Solvens wird im Vakuum entfernt und das Hydrochlorid im Hochvakuum getrocknet.

### Allgemeine Vorschrift zur Synthese der Ausführungsbeispiele 5 und 6:

1.0 eq. des Beispiels 6A, 1.0 eq. der entsprechenden Boronsäure, 0.05 eq. 1,1'-Bis(diphenylphosphino)ferrocenpalladium(II)chlorid und 3.0 eq. 2 *M* Natriumcarbonat-Lösung werden in Dimethylformamid für 14 h auf 80°C erhitzt. Nach der Filtration über Kieselgur erfolgt die Reinigung der Produkte chromatographisch durch präparative HPLC. Das aufgereinigte Produkt wird in Methanol gelöst und mit 4 *M* HCl in Dioxan versetzt. Das Solvens wird im Vakuum entfernt und das Hydrochlorid im Hochvakuum getrocknet.

### Beispiel 1

### N-[4-(2-Thienyl)phenyl]-1-azabicyclo[2.2.2]octan-3-carboxamid-Hydrochlorid

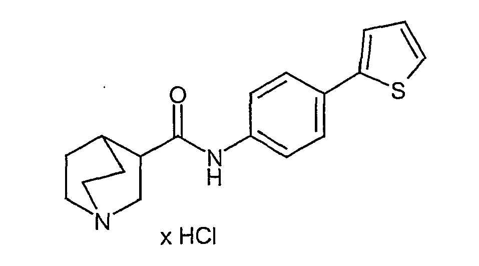

90 mg (0.26 mmol) N-(4-Bromphenyl)-1-azabicyclo[2.2.2]octan-3-carboxamid-Hydrochlorid, 40 mg (0.31 mmol) 2-Thiophenboronsäure, 190 mg (0.57 mmol) Caesiumcarbonat und 20 mg (0.03 mmol) Bis(diphenylphosphino)ferrocenpalladium(II)chlorid werden in 1 mL 1,2-Dimethoxyethan gemäß der allgemeinen Arbeitsvorschrift umgesetzt. Es werden 14.1 mg (15% d.Th.) der Titelverbindung erhalten.
LC-MS (Methode A): Rₜ = 2.80 min., MS (ESIpos): *m*/*z* = 313 (M+H)⁺ (freie Base).

### Beispiel 2

### N-[4'-(Hydroxymethyl)-1,1'-biphenyl-4-yl]-1-azabicyclo[2.2.2]octan-3-carboxamid-Hydrochlorid

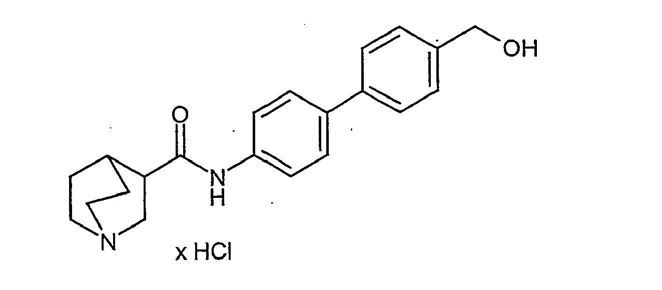

90 mg (0.26 mmol) N-(4-Bromphenyl)-1-azabicyclo[2.2.2]octan-3-carboxamid-Hydrochlorid, 50 mg (0.31 mmol) 4-(Hydroxymethyl)phenylboronsäure, 190 mg (0.57 mmol) Caesiumcarbonat und 20 mg (0.03 mmol) Bis(diphenylphosphino)-ferrocenpalladium(II)chlorid werden in 1 mL 1,2-Dimethoxyethan gemäß der allgemeinen Arbeitsvorschrift umgesetzt. Es werden 5.9 mg (6% d.Th.) der Titelverbindung erhalten.
LC-MS (Methode A): Rₜ = 2.40 min., MS (ESIpos): *m*/*z* = 337 (M+H)⁺ (freie Base).

### Beispiel 3

### N-(4'-Fluor-1,1'-biphenyl-4-yl)-1-azabicyclo[2.2.2]octan-3-carboxamid-Hydrochlorid

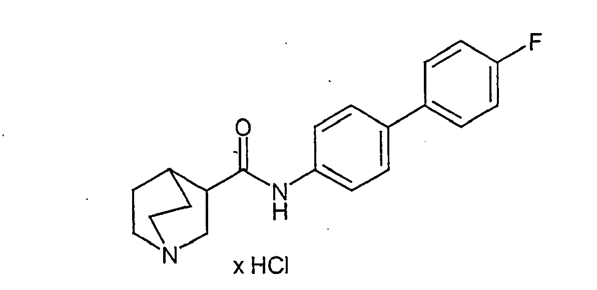

90 mg (0.26 mmol) N-(4-Bromphenyl)-1-azabicyclo[2.2.2]octan-3-carboxamid-Hydrochlorid, 40 mg (0.31 mmol) 4-Fluorphenylboronsäure, 190 mg (0.57 mmol) Caesiumcarbonat und 20 mg (0.03 mmol) Bis(diphenylphosphino)ferrocenpalladium-(II)chlorid werden in 1 mL 1,2-Dimethoxyethan gemäß der allgemeinen Arbeitsvorschrift umgesetzt. Es werden 17.6 mg (19% d.Th.) der Titelverbindung erhalten.
¹H-NMR (200 MHz, DMSO-d₆): δ = 10.38 (breites s, 1H), 9.83 (breites s, 1H), 7.76-7.57 (m, 6H), 7.33-7.20 (m, 2H), 3.70-3.10 (m, 8H), 2.00-1.65 (m, 4H).
LC-MS (Methode B): Rₜ = 2.88 min., MS (ESIpos): *m*/*z* = 325 (M+H)⁺ (freie Base).

### Beispiel 4

### N-(4'-Methylsulfanyl-1,1'-biphenyl-4-yl)-1-azabicyclo[2.2.2]octan-3-carboxamid-Hydrochlorid

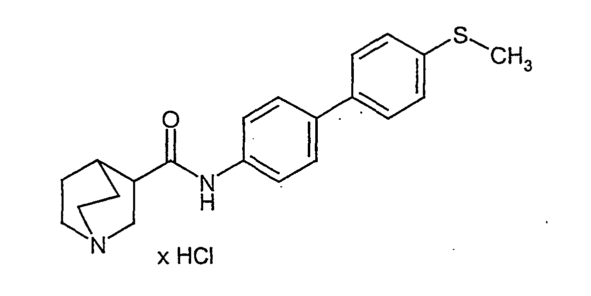

90 mg (0.26 mmol) N-(4-Bromphenyl)-1-azabicyclo[2.2.2]octan-3-carboxamid-Hydrochlorid, 50 mg (0.31 mmol) 4-(Methylsulfanyl)phenylboronsäure, 190 mg (0.57 mmol) Caesiumcarbonat und 20 mg (0.03 mmol) Bis(diphenylphosphino)-ferrocenpalladium(II)chlorid werden in 1 mL 1,2-Dimethoxyethan gemäß der allgemeinen Arbeitsvorschrift umgesetzt. Es werden 21.6 mg (21% d.Th.) der Titelverbindung erhalten.
LC-MS (Methode B): Rₜ = 3.01 min., MS (ESIpos): *m*/*z* = 353 (M+H)⁺ (freie Base).

### Beispiel 5

### 2-(1-Azabicyclo[2.2.2]oct-3-yl)-N-(4'-fluor-1,1'-biphenyl-4-yl)acetamid-Hydrochlorid

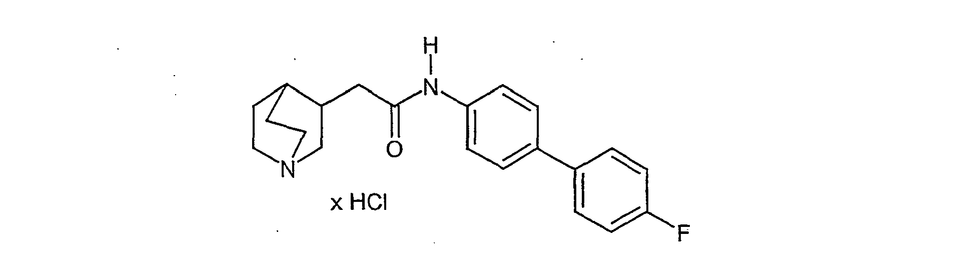

Entsprechend der allgemeinen Vorschrift werden 60 mg (0.17 mmol) 2-(1-Azabicyclo[2.2.2]oct-3-yl)-N-(4-bromphenyl)acetamid-Hydrochlorid, 23.3 mg (0.17 mmol) 4-Fluorphenylboronsäure, 0.17 mL 2 *M* Natriumcarbonat-Lösung und 6.1 mg (0.01 mmol) Bis(diphenylphosphino)ferrocenpalladium(II)chlorid in 1 mL Dimethylformamid umgesetzt. Es werden 32 mg (51% d.Th.) der Titelverbindung erhalten.
¹H-NMR (200 MHz, DMSO-d₆): δ = 10.27 (s, 1H), 9.81 (s, 1H), 7.75-7.56 (m, 6H), 7.27 (m, 2H), 3.55-3.30 (m), 3.21 (m, 4H), 2.92 (dd, 1H), 2.76-2.40 (m), 2.05-1.61 (m, 5H).
MS (ESIpos): *m*/*z* = 339 (M+H)⁺ (freie Base)
HPLC (Kromasil RP-18, 60 x 2.1 mm; Eluent A: H₂O + 5 mL HClO₄/L, Eluent B: Acetonitril; Gradient: 0 - 4.5 min 98% A → 90% B, 4.5 - 6.5 min 90% B; Fluss: 0.75 mL/min; Temp.: 30°C; UV-Detektion bei 210 nm): Rₜ = 4.20 min.

### Beispiel 6

### 2-(1-Azabicyclo[2.2.2]oct-3-yl)-N-(4'-methoxy-1,1'-biphenyl-4-yl)acetamid-Hydrochlorid

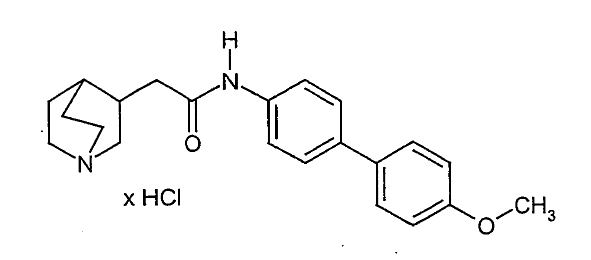

Entsprechend der allgemeinen Vorschrift werden 60 mg (0.17 mmol) 2-(1-Azabicyclo[2.2.2]oct-3-yl)-N-(4-bromphenyl)acetamid-Hydrochlorid, 25.4 mg (0.17 mmol) 4-Methoxyphenylboronsäure, 0.17 mL 2 *M* Natriumcarbonat-Lösung und 6.1 mg (0.01 mmol) Bis(diphenylphosphino)ferrocenpalladium(II)chlorid in 1 mL Dimethylformamid umgesetzt. Es werden 34 mg (50% d.Th.) der Titelverbindung erhalten. MS (ESIpos): *m*/*z* = 351 (M+H)⁺ (freie Base)
HPLC (Kromasil RP-18, 60 x 2.1 mm; Eluent A: H₂O + 5 mL HClO₄/L, Eluent B: Acetonitril; Gradient: 0 - 4.5 min 98% A → 90% B, 4.5 - 6.5 min 90% B; Fluss: 0.75 mL/min; Temp.: 30°C; UV-Detektion bei 210 nm): Rₜ = 4.10 min.

### Beispiel 7

### 2-(1-Azabicyclo[2.2.2]oct-3-yl)-N-(4'-fluor-1,1'-biphenyl-3-yl)acetamid-Hydrochlorid

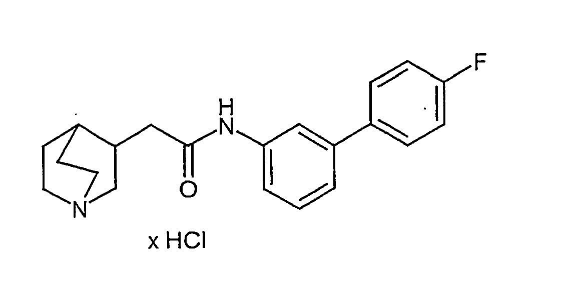

Zu einer Lösung von 75 mg (0.16 mmol) 2-(1-Azabicyclo[2.2.2]oct-3-yl)-N-(3-bromphenyl)acetamid-Hydrochlorid in 1 mL DMF werden 22.1 mg (0.16 mmol) 4-Fluorphenylboronsäure, 0:17 mL (0.34 mmol) 2 M wässrige Natriumcarbonat-Lösung und 5.8 mg (0.01 mmol) 1,1'-Bis(diphenylphosphino)ferrocenpalladium(II)-chlorid zugegeben. Das Reaktionsgemisch wird über Nacht bei 80°C gerührt. Es werden nochmals 22.1 mg (0.16 mmol) 4-Fluorphenylboronsäure, 23.2 mg (0.03 mmol) 1,1'-Bis(diphenylphosphino)ferrocenpalladium(II)chlorid und 0.48 mL (0.48 mmol) 1 M Natronlauge zugegeben. Anschließend wird für weitere 12 h auf 80°C erhitzt. Nach Reaktionsende wird abgekühlt, über Kieselgel filtriert und anschließend mittels präparativer HPLC gereinigt. Die Produkt-Fraktionen werden eingeengt, in Methanol aufgenommen, mit 4 M HCl in Dioxan versetzt und erneut eingeengt. Nach. Trocknung im Hochvakuum erhält man 49.6 mg (83.4% d.Th.) der Titelverbindung.
HPLC (Methode E): Rₜ = 4.2 min.
MS (DCI): *m*/*z* = 339 (M+H)⁺ (freie Base).

### Beispiel 8

### 2-(1-Azabicyclo[2.2.2]oct-3-yl)-N-(3'-nitro-1,1'-biphenyl-4-yl)acetamid-Hydrochlorid

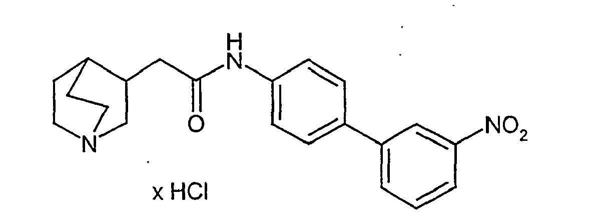

Zu einer Lösung von 120 mg (0.33 mmol) 2-(1-Azabicyclo[2.2.2]oct-3-yl)-N-(4-bromphenyl)acetamid-Hydrochlorid in 2 mL DMF werden 61.3 mg (0.37 mmol) 3-Nitrophenylboronsäure, 0.5 mL (1.0 mmol) 2 M wässrige Natriumcarbonat-Lösung und 12.2 mg (0.02 mmol) 1,1'-Bis(diphenylphosphino)ferrocenpalladium(II)chlorid zugegeben. Das Reaktionsgemisch wird 14 h bei 80°C gerührt, abgekühlt, über Kieselgur filtriert und anschließend mittels präparativer HPLC gereinigt. Die Produkt-Fraktionen werden eingeengt, in einem 5:1-Gemisch aus Methanol und 1 M Salzsäure aufgenommen und erneut eingeengt. Nach Trocknung im Hochvakuum erhält man 13 mg (9.7% d.Th.) der Titelverbindung.
HPLC (Methode E): Rₜ = 4.1 min.
MS (DCI): *m*/*z* = 366 (M+H)⁺ (freie Base).

### Beispiel 9

### 2-(1-Azabicyclo[2.2.2]oct-3-yl)-N-[4'-(hydroxymethyl)-1,1'-biphenyl-3-yl]acetamid-Hydrochlorid

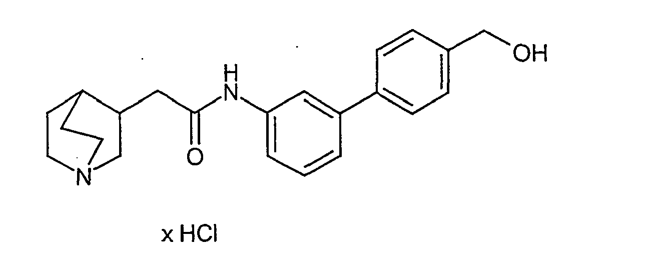

Zu einer Lösung von 75 mg (0.16 mmol) 2-(1-Azabicyclo[2.2.2]oct-3-yl)-N-(3-bromphenyl)acetamid-Hydrochlorid in 1 mL DMF werden 24.0 mg (0.16 mmol) 4-(Hydroxymethyl)phenylboronsäure, 0.17 mL (0.34 mmol) 2 M wässrige Natriumcarbonat-Lösung und 5.8 mg (0.01 mmol) 1,1'-Bis(diphenylphosphino)ferrocen-palladium(II)chlorid zugegeben. Das Reaktionsgemisch wird 14 h bei 80°C gerührt. Es werden weitere 24.0 mg (0.16 mmol) 4-(Hydroxymethyl)phenylboronsäure, 23.2 mg (0.03 mmol) 1,1'-Bis(diphenylphosphino)ferrocenpalladium(II)chlorid und 0.48 ml (0.48 mmol) 1 M Natronlauge zugegeben. Anschließend wird für weitere 12 h auf 80°C erhitzt. Nach Reaktionsende wird abgekühlt, über Kieselgel filtriert und anschließend mittels präparativer HPLC gereinigt. Die Produkt-Fraktionen werden eingeengt, in Methanol aufgenommen, mit 4 M HCl in Dioxan versetzt und erneut eingeengt. Nach Trocknung im Hochvakuum erhält man 25.9 mg (39.8% d.Th.) der Titelverbindung.
HPLC (Methode E): Rₜ = 3.7 min.
MS (DCI): *m*/*z* = 351 (M+H)⁺ (freie Base).

### Beispiel 10

### 2-(1-Azabicyclo[2.2.2]oct-3-yl)-N-[4'-(brommethyl)-1,1'-biphenyl-4-yl]acetamid-Hydrochlorid

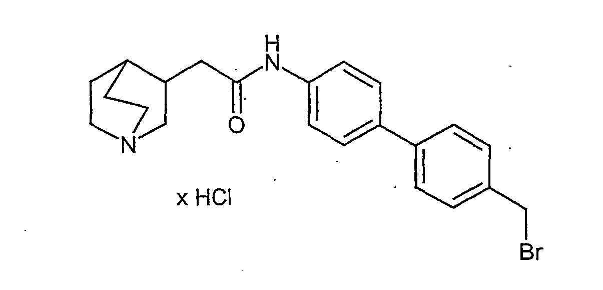

Zu einer Lösung von 100 mg (0.28 mmol) 2-(1-Azabicyclo[2.2.2]oct-3-yl)-N-(4-bromphenyl)acetamid-Hydrochlorid in 1.5 mL DMF werden 59.7 mg (0.28 mmol) 4-(Brommethyl)phenylboronsäure, 0.17 mL (0.34 mmol) 2 M wässrige Natriumcarbonat-Lösung und 10.7 mg (0.01 mmol) 1,1'-Bis(diphenylphosphino)ferrocen-palladium(II)chlorid zugegeben. Das Reaktionsgemisch wird 14 h lang bei 80°C gerührt, abgekühlt, über Kieselgel filtriert und anschließend mittels präparativer HPLC gereinigt. Die Produkt-Fraktionen werden eingeengt, in Methanol aufgenommen, mit 4 M HCl in Dioxan versetzt und erneut eingeengt. Nach Trocknung im Hochvakuum erhält man 20 mg (16% d.Th.) der Titelverbindung.
HPLC (Methode E): Rₜ = 4.6 min.
MS (ESIpos): *m*/*z* = 413 (M+H)⁺ (freie Base).

### Beispiel 11

### 2-(1-Azabicyclo[2.2.2]oct-3-yl)-N-[2'-(hydroxymethyl)-1,1'-biphenyl-3-yl]acetamid-Hydrochlorid

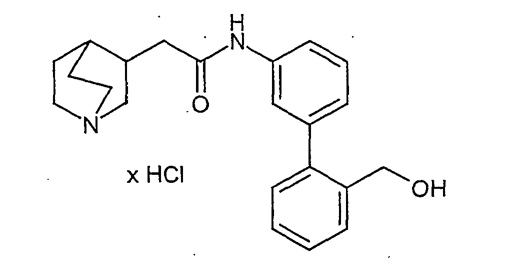

Zu einer Lösung von 150 mg (0.32 mmol) 2-(1-Azabicyclo[2.2.2]oct-3-yl)-N-(3-bromphenyl)acetamid-Hydrochlorid in 2 mL DMF werden 48.1 mg (0.32 mmol) 2-(Hydroxymethyl)phenylboronsäure, 0.95 mL (0.95 mmol) 1 M Natronlauge und 51.7 mg (0.06 mmol) 1,1'-Bis(diphenylphosphino)ferrocenpalladium(II)chlorid zugegeben. Das Reaktionsgemisch wird 18 h lang bei 80°C gerührt. Es werden nochmals die gleichen Mengen an 2-(Hydroxymethyl)phenylboronsäure, 1,1'-Bis(diphenylphosphino)ferrocenpalladium(II)chlorid und Natronlauge zugegeben und weitere 24 h auf 80°C erhitzt. Das Reaktionsgemisch wird abgekühlt, über Kieselgur filtriert und anschließend mittels präparativer HPLC gereinigt. Die Produkt-Fraktionen werden eingeengt, in Methanol aufgenommen, mit 4 M HCl in Dioxan versetzt und erneut eingeengt. Nach Trocknung im Hochvakuum erhält man 86.5 mg (64.1% d.Th.) der Titelverbindung.
HPLC (Methode E): Rₜ = 4.3 min.
LC-MS (Methode D): *m*/*z* = 351 (M+H)⁺ (freie Base), Rₜ = 2.6 min.

### Beispiel 12

### N-[3'-(Acetylamino)-1,1'-biphenyl-4-yl]-2-(1-azabicyclo[2.2.2]oct-3-yl)acetamid-Hydrochlorid

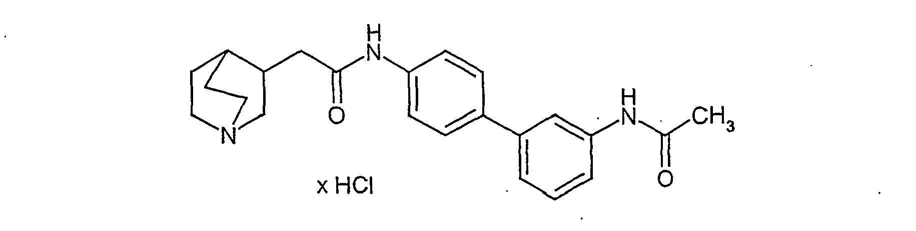

Zu einer Lösung von 80 mg (0.22 mmol) 2-(1-Azabicyclo[2.2.2]oct-3-yl)-N-(4-bromphenyl)acetamid-Hydrochlorid in 1.5 mL DMF werden 43.8 mg (0.24 mmol) 3-(Acetamido)phenylboronsäure, 0.33 mL (0.66 mmol) 2 M wässrige Natriumcarbonat-Lösung und 8.1 mg (0.01 mmol) 1,1'-Bis(diphenylphosphino)ferrocenpalladium(II)-chlorid zugegeben. Das Reaktionsgemisch wird 14 h lang bei 80°C gerührt, abgekühlt, über Kieselgur filtriert und anschließend mittels präparativer HPLC gereinigt. Die Produkt-Fraktionen werden eingeengt, in Methanol aufgenommen, mit 4 M HCl in Dioxan versetzt und erneut eingeengt. Nach Trocknung im Hochvakuum erhält man 23 mg (20% d.Th.) der Titelverbindung.
HPLC (Methode E): Rₜ = 3.6 min.
MS (ESIpos): *m*/*z* = 378 (M+H)⁺ (freie Base).

### Beispiel 13

### (3R)-N-[2'-(Hydroxymethyl)-1,1'-biphenyl-4-yl]-1-azabicyclo[2.2.2]octan-3-carboxamid

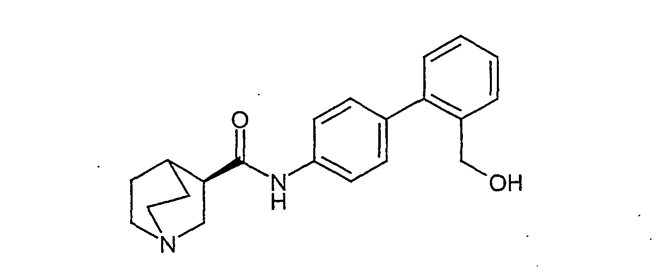

Eine Mischung aus 90 mg (0.58 mmol) 2-(Hydroxymethyl)phenylboronsäure, 120 mg (0.39 mmol) (3R)-N-(4-Bromphenyl)-1-azabicyclo[2.2.2]octan-3-carboxamid, 1.16 mL (1.16 mmol) 1 N Natronlauge, 30 mg (0.04 mmol) 1,1'-Bis(diphenylphosphino)ferrocenpalladium(II)chlorid und 1 mL DMF wird 42 h lang auf 80-85°C erhitzt. Das Solvens wird unter reduziertem Druck entfernt. Das Rohprodukt wird über Kieselgel 60 (Laufmittel: Dichlormethan → Dichlormethan/Methanol/Ammoniak 80:20:2) chromatographisch gereinigt. Es werden 56 mg (39% d.Th.) der Titelverbindung erhalten.
¹H-NMR (400 MHz, DMSO-d₆): δ = 10.06 (s, 1H), 8.29 (s, 1H), 7.65 (d, 2H), 7.55 (d, 1H), 7.40-7.12 (m, 4H), 4.41 (s, 2H), 3.41-3.22 (m, 1H), 3.03-2.70 (m, 6H), 2.22-2.15 (m, 1H), 1.77-1.58 (m, 3H), 1.52-1.38 (m, 1H).
HPLC (Methode E): Rₜ = 3.69 min.
LC-MS (Methode B): Rₜ = 2.47 min., MS (ESIpos): *m*/*z* = 337 (M+H)⁺.

### Beispiel 14

### (3R)-N-[4'-(Hydroxymethyl)-1,1'-biphenyl-4-yl]-1-azabicyclo[2.2.2]octan-3-carboxamid-Hydrochlorid

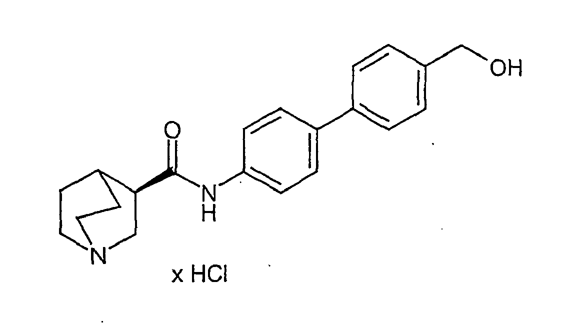

Eine Mischung aus 90 mg (0.58 mmol) 4-(Hydroxymethyl)phenylboronsäure, 120 mg (0.39 mmol) (3*R*)-N-(4-Bromphenyl)-1-azabicyclo[2.2.2]octan-3-carboxamid, 1.16 mL (1.16 mmol) 1 N Natronlauge, 30 mg (0.04 mmol) 1,1'-Bis(diphenylphosphino)ferrocenpalladium(II)chlorid und 1 mL DMF wird 42 h lang auf 80-85°C erhitzt. Das Solvens wird unter reduziertem Druck entfernt. Das Rohprodukt wird über Kieselgel 60 (Laufmittel: Dichlormethan → Dichlormethan/Methanol/Ammoniak 80:20:2) chromatographisch gereinigt. Eine Feinreinigung mittels präparativer HPLC schließt sich an. Das Produkt wird in Methanol gelöst und mit einem Überschuß an HCl in Diethylether versetzt. Das Solvens wird unter reduziertem Druck abgezogen und letzte Lösungsmittelreste im Hochvakuum entfernt. Es werden 68 mg (47% d.Th.) der Titelverbindung erhalten.
¹H-NMR (300 MHz, DMSO-d₆): δ =10.45 (s, 1H), 10.25 (s, 1H), 7.73-7.52 (m, 6H), 7.40-7.32 (m, 2H), 5.15 (s, 1H), 4.52 (s, 2H), 3.63-3.52 (m, 1H), 3.42-3.00 (m, 7H), 1.98-1.88 (m, 2H), 1.80-1.68 (m, 2H).
HPLC (Methode E): Rₜ = 3.54 min.
MS (ESIpos): *m*/*z* = 337 (M+H)⁺.

### Beispiel 15

### (3S)-N-[4'-(Hydroxymethyl)-1,1'-biphenyl-4-yl]-1-azabicyclo[2.2.2]octan-3-carboxamid-Hydrochlorid

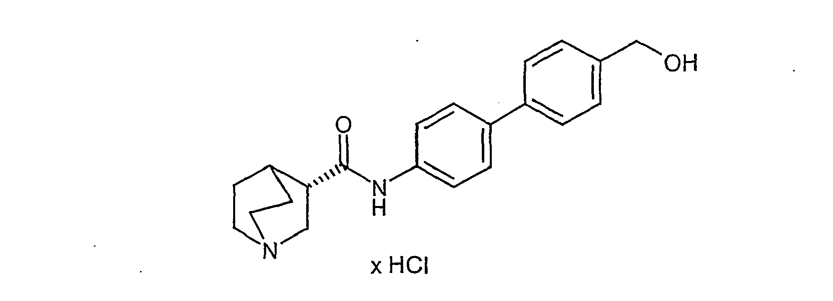

Eine Mischung aus 90 mg (0.58 mmol) 4-(Hydroxymethyl)phenylboronsäure, 120 mg (0.39 mmol) (3*S*)-N-(4-Bromphenyl)-1-azabicyclo[2.2.2]octan-3-carboxamid, 1.16 mL (1.16 mmol) 1 N Natronlauge, 30 mg (0.04 mmol) 1,1'-Bis(diphenylphosphino)ferrocenpalladium(II)chlorid und 1 mL DMF wird 42 h lang auf 80-85°C erhitzt. Das Solvens wird unter reduziertem Druck entfernt. Das Rohprodukt wird über Kieselgel 60 (Laufmittel: Dichlormethan → Dichlormethan/Methanol/Ammoniak 80:20:2) chromatographisch gereinigt. Eine Feinreinigung mittels präparativer HPLC schließt sich an. Das Produkt wird in Methanol gelöst und mit einem Überschuß an HCl in Diethylether versetzt. Das Solvens wird unter reduziertem Druck abgezogen und letzte Lösungsmittelreste im Hochvakuum entfernt. Es werden 37 mg (26% d.Th.) der Titelverbindung erhalten.
Die analytischen Daten stimmen mit denen des R-Enantiomers (Beispiel 14) überein.

### Beispiel 16

### (3R)-N-[4'-(4-Morpholinyl)-1,1'-biphenyl-4-yl]-1-azabicyclo[2.2.2]octan-3-carboxamid

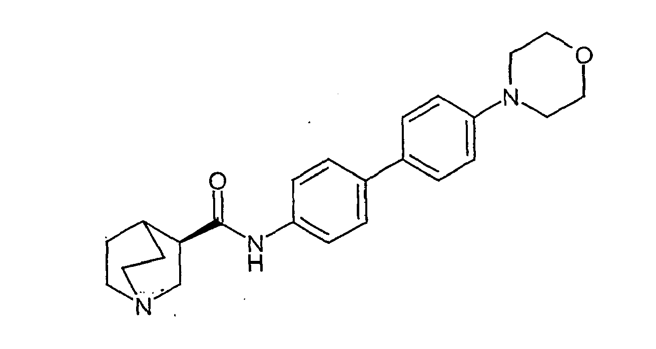

Eine Mischung aus 120 mg (0.58 mmol) 4-Morpholinophenylboronsäure, 120 mg (0.39 mmol) (3*R*)-N-(4-Bromphenyl)-1-azabicyclo[2.2.2]octan-3-carboxamid, 1.16 mL (1.16 mmol) 1 N Natronlauge, 30 mg (0.04 mmol) 1,1'-Bis(diphenylphosphino)-ferrocenpalladium(II)chlorid und 1 mL DMF wird 40 h lang auf 80-85°C erhitzt. Das Solvens wird unter reduziertem Druck entfernt. Das Rohprodukt wird über Kieselgel 60 (Laufmittel: Dichlormethan → Dichlormethan/Methanol/Ammoniak 80:20:2) chromatographisch gereinigt. Das Solvens wird unter reduziertem Druck abgezogen und letzte Lösungsmittelreste im Hochvakuum entfernt. Es werden 100 mg (66% d.Th.) der Titelverbindung erhalten.
¹H-NMR (400 MHz, DMSO-d₆): δ = 10.11 (s, 1H), 8.22 (s, 1H), 7.67-7.45 (m, 6H), 6.97 (m, 2H), 3.75 (m, 4H), 3.45-3.37 (m, 1H), 3.15-2.75 (m, 7H), 2.77 (m, 4H), 1.80-1.62 (m, 3H), 1.58-1.48 (m, 1H).
HPLC (Methode E): Rₜ = 3.42 min.
MS (DCI / NH₃): *m*/*z* = 392 (M+H)⁺.

### Beispiel 17

### (3R)-N-[4'-(Hydroxymethyl)-3'-(methoxy)-1,1'-biphenyl-4-yl]-1-azabicyclo[2.2.2]-octan-3-carboxamid

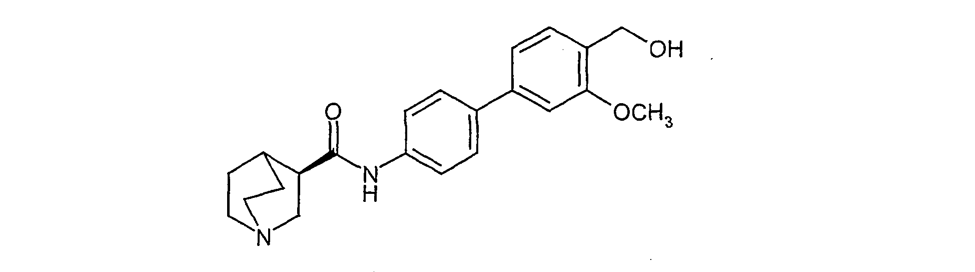

118 mg (0.47) Bis(pinacolato)dibor, 193 mg (1.4 mmol) trockenes Kaliumcarbonat, und (4-Brom-2-methoxyphenyl)methanol werden in 1 mL DMF gelöst. Es wird für 15 Minuten Argon durch das Reaktionsgemisch geleitet, anschließend mit 14.2 mg (0.02 mmol) PdCl₂(dppf) versetzt und über Nacht auf 85°C erhitzt. Anschließend werden 120 mg (0.39 mmol) (3*R*)-N-(4-Bromphenyl)-1-azabicyclo[2.2.2]octan-3-carboxamid, 1.94 mL 1 N Natronlauge und weitere 14.2 mg (0.02 mmol) PdCl₂(dppf) zugegeben. Es wird über Nacht auf 85°C erhitzt. Das Solvens wird unter reduziertem Druck entfernt. Das Rohprodukt wird über Kieselgel 60 (Laufmittel: Dichlormethan → Dichlormethan/Methanol/Ammoniak 60:40:2) chromatographisch gereinigt. Das Solvens wird unter reduziertem Druck abgezogen und letzte Lösungsmittelreste im Hochvakuum entfernt. Es werden 5 mg (4% d.Th.) der Titelverbindung erhalten.
¹H-NMR (200 MHz, DMSO-d₆): δ = 10.38 (s, 1H), 7.80-7.10 (m, 7H), 5.03 (m, 1H), 4.51 (s, 2H), 3.85 (s, 3H), 3.70-2.95 (m, 8H), 2.01-1.63 (m, 4H).
HPLC (Methode E): Rₜ = 3.64 min.
MS (ESIpos): *m*/*z* = 367 (M+H)⁺.

### Beispiel 18

### 4'-{[(3S)-1-Azabicyclo[2.2.2]oct-3-ylcarbonyl]amino}-1,1'-biphenyl-4-carbonsäuremethylester

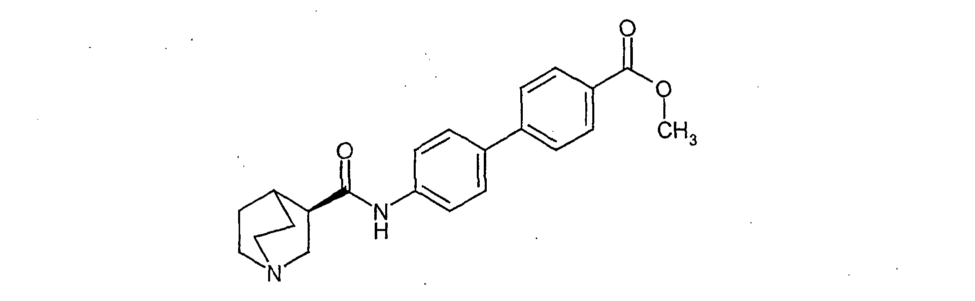

679 mg (3.91 mmol) (3*R*)-1-Azabicyclo[2.2.2]octan-3-carbonylchlorid, 846 mg (3.73 mmol) 4'-Amino-1,1'-biphenyl-4-carbonsäuremethylester, 963 mg (7.45 mmol) N,N-Diisopropylethylamin sowie 227 mg (1.86 mmol) 4-N,N-Dimethylaminopyridin werden in 5 mL THF gelöst und über Nacht bei RT, dann nochmals über Nacht bei 50°C gerührt. Das Reaktionsgemisch wird in Dichlormethan und Wasser aufgenommen und die wässrige Phase dreimal mit Dichlormethan extrahiert. Das Rohprodukt wird über Kieselgel 60 (Laufmittel: Dichlormethan/Triethylamin 100:1 → Dichlormethan/Methanol/Triethylamin 50:50:1) chromatographisch gereinigt. Das Solvens wird unter reduziertem Druck entfernt. Das Produkt wird in 1 N Natronlauge aufgenommen und insgesamt dreimal mit Essigsäureethylester extrahiert. Die organische Phase wird über Magnesiumsulfat getrocknet und unter reduziertem Druck vom Solvens befreit. Schließlich werden letzte Lösungsmittelreste im Hochvakuum entfernt. Es werden 50 mg (4% d.Th.) der Titelverbindung erhalten.
¹H-NMR (300 MHz, DMSO-d₆): δ = 10.00 (s, 1H), 8.05-7.63 (m, 8H), 3.85 (s, 3H), 3.40-2.65 (m, 8H), 1.70-1.25 (m, 4H).
HPLC (Methode E): Rₜ = 4.11 min.
MS (ESIpos): *m*/*z* = 365 (M+H)⁺.

### Beispiel 19

### 4'-{[(3S)-1-Azabicyclo[2.2.2]oct-3-ylcarbonyl]amino}-1,1'-biphenyl-4-carbonsäureHydrochlorid

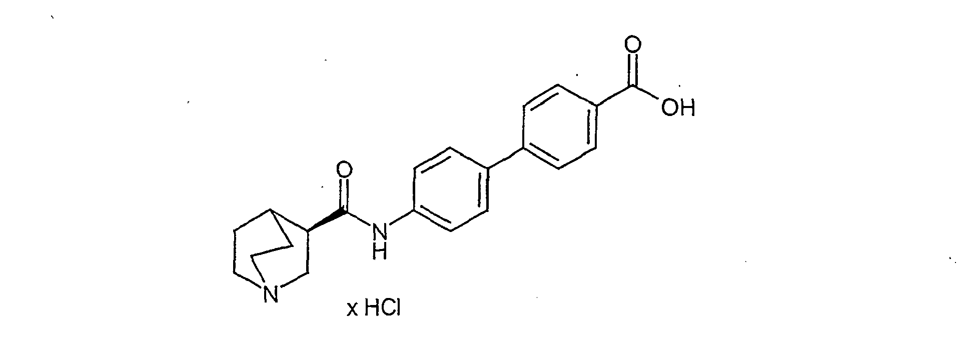

100 mg (0.27 mmol) 4'-{[(3*S*)-1-Azabicyclo[2.2.2]oct-3-ylcarbonyl]amino}-1,1'-biphenyl-4-carbonsäuremethylester werden in 2 mL Methanol suspendiert. Es werden 200 mg (3.54 mmol) Kaliumhydroxid und einige Tropfen Wasser hinzugegeben. Es wird über Nacht unter Rückfluss erhitzt. Das Solvens wird unter reduziertem Druck entfernt. Der Rückstand wird mit 1 N Salzsäure versetzt, wobei das Produkt ausfällt. Dieses wird abfiltriert und mit wenig Wasser gewaschen. Es werden 60 mg (57% d.Th.) der Titelverbindung erhalten.
¹H-NMR (200 MHz, DMSO-d₆): δ = 10.50 (s, 1H), 9.95 (s, 1H), 8.05-7.63 (m, 8H), 3.70-3.05 (m, 8H), 2.05-1.65 (m, 4H).
HPLC (Methode E): Rₜ = 3.56 min.
MS (ESIpos): *m*/*z* = 351 (M+H)⁺.

### Beispiel 20

### (3R)-N-[4'-(1-Hydroxy-1-methylethyl)-1,1'-biphenyl-4-yl]-1-azabicyclo[2.2.2]octan-3-carboxamid

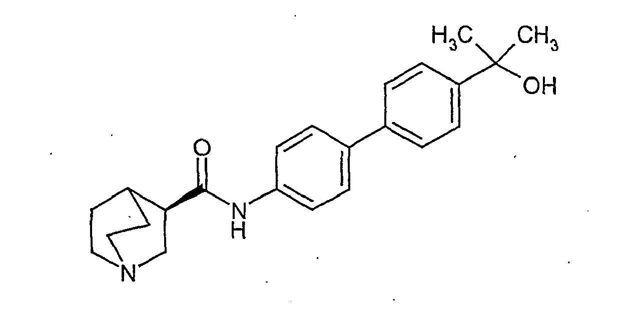

5.75 mL (8.05 mmol) Methylmagnesiumbromid (1.6 M Lösung in 1:1 THF/Toluol) werden bei 0°C unter Argon mit 230 mg (0.63 mmol) 4'-{[(3*S*)-1-Azabicyclo[2.2.2]-oct-3-ylcarbonyl]amino}-1,1'-biphenyl-4-carbonsäuremethylester, suspendiert in 1 mL THF, versetzt. Das Reaktionsgemisch wird über Nacht bei RT gerührt. Unter Eiskühlung wird 1 N Natronlauge zugegeben und fünfmal mit Essigsäureethylester extrahiert. Die vereinigten organischen Phasen werden über Magnesiumsulfat getrocknet. Das Rohprodukt wird über Kieselgel 60 (Laufmittel: Dichlormethan/Methanol/Ammoniak 90:10:1 → 80:20:1) gereinigt. Das Solvens wird unter reduziertem Druck entfernt. Es werden 193 mg (81% d.Th.) der Titelverbindung erhalten.
HPLC (Methode E): Rₜ = 3.82 min.
MS (ESIpos): *m*/*z* = 365 (M+H)⁺.

### Beispiel 21

### (3R)-N-[4'-(Aminocarbonyl)-1,1'-biphenyl-4-yl]-1-azabicyclo[2.2.2]octan-3-carboxamid-Hydrochlorid

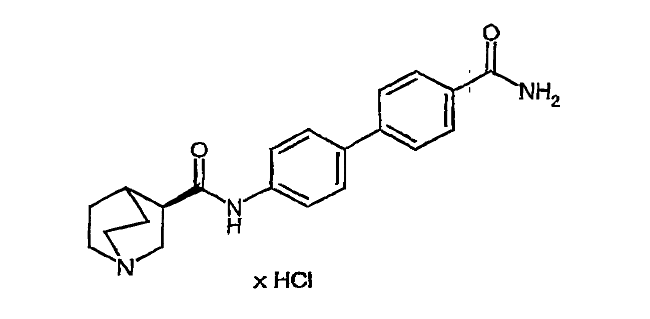

60 mg (0.16 mmol) 4'-{[(3*S*)-1-Azabicyclo[2.2.2]oct-3-ylcarbonyl]amino}-1,1'-biphenyl-4-carbonsäure-Hydrochlorid und 2 mL (27.4 mmol) Thionylchlorid werden 3 h unter Rückfluss erhitzt. Der Überschuss an Thionylchlorid wird abdestilliert. Das so hergestellte rohe Säurechlorid wird in 1 mL THF vorgelegt und mit 3.1 mL (1.55 mmol) Ammoniak (0.5 M Lösung in Dioxan) drei Tage lang bei RT gerührt. Das Solvens wird unter reduziertem Druck entfernt und das Rohprodukt mittels präparativer HPLC gereinigt. Es werden 11 mg (18% d.Th.) der Titelverbindung erhalten.
HPLC (Methode E): Rₜ = 3.30 min.
MS (ESIpos): *m*/*z =* 350 (M+H)⁺.

### Beispiel 22

### (3R)-N-[4'-Hydroxymethyl)-3'-fluor-1,1'-biphenyl-4-yl]-1-azabicyclo[2.2.2]octan-3-carboxamid

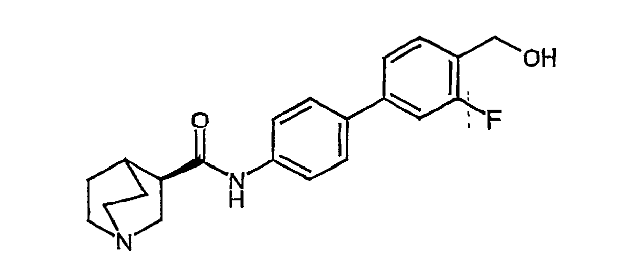

492 mg (1.62) Bis(pinacolato)dibor, 804 mg (5.82 mmol) trockenes Kaliumcarbonat, und 398 mg (1.94 mmol) (4-Brom-2-fluorphenyl)methanol werden in 4.2 mL DMF gelöst. Es wird für 15 Minuten Argon durch das Reaktionsgemisch geleitet, anschließend mit 59 mg (0.08 mmol) PdCl₂(dppf) versetzt und über Nacht auf 85°C erhitzt. Anschließend werden 500 mg (1.62 mmol) (3*R*)-N-(4-Bromphenyl)-1-azabicyclo[2.2.2]octan-3-carboxamid, 8.1 mL 1 N Natronlauge und weitere 59 mg (0.02 mmol) PdCl₂(dppf) zugegeben. Es wird über Nacht auf 85°C erhitzt. Das Solvens wird unter reduziertem Druck entfernt. Das Rohprodukt wird mittels präparativer HPLC gereinigt. Es werden 21 mg (3% d.Th.) der Titelverbindung erhalten.
¹H-NMR (400 MHz, CD₃OD): δ = 8.52 (s, 1H), 7.70-7.25 (m, 7H), 4.51 (s, 2H), 3.92-3.75 (m, 1H), 3.50-3.10 (m, 6H), 2.58-2.46 (m, 1H), 2.21-1.94 (m, 3H), 1.93-1.76 (m, 1H). HPLC (Methode E): Rₜ = 3.66 min.
MS (ESIpos): *m*/*z* = 355 (M+H)⁺.

### Beispiel 23

### (4'-{[(3R)-1-Azabicyclo[2.2.2]oct-3-ylcarbonyl]amino}-1,1'-biphenyl-4-yl)methylmethylcarbamat

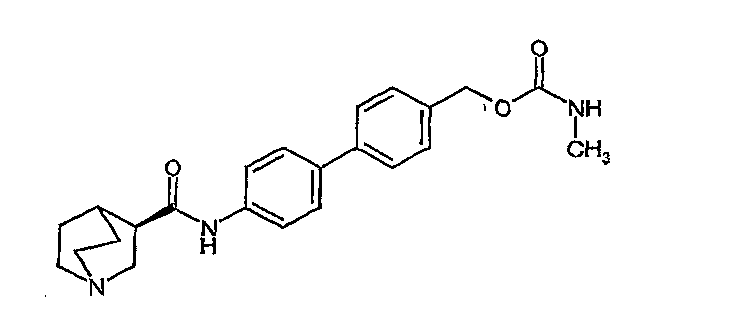

35 mg (0.10 mmol) (3*R*)-N-[4'-(Hydroxymethyl)-1,1'-biphenyl-4-yl]-1-azabicyclo-[2.2.2]octan-3-carboxamid werden in 1 mL einer 1:1-Mischung aus THF/DMF gelöst. Es werden 12 mg (0.21 mmol) Methylisocyanat zugegeben und über Nacht bei 60°C gerührt. Das Solvens wird unter reduziertem Druck entfernt und das Rohprodukt mittels präparativer HPLC gereinigt. Es werden 20 mg (49% d.Th.) der Titelverbindung erhalten.
HPLC (Methode E): Rₜ = 3.80 min.
MS (ESIpos): *m*/*z* = 394 (M+H)⁺.

### Beispiel 24

### (4'-{[(3R)-1-Azabicyclo[2.2.2]oct-3-ylcarbonyl]amino}-1,1'-biphenyl-4-yl)methylisopropylcarbamat

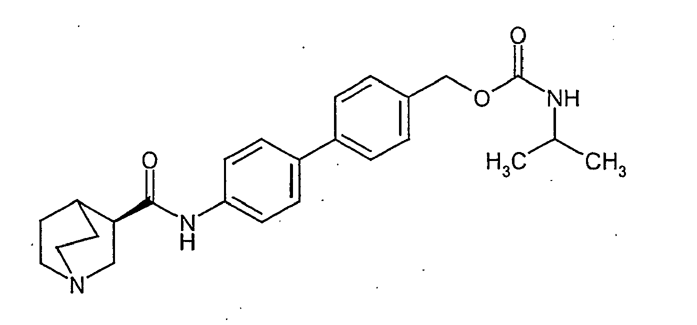

35 mg (0.10 mmol) (3*R*)-N-[4'-(Hydroxymethyl)-1,1'-biphenyl-4-yl]-1-azabicyclo-[2.2.2]octan-3-carboxamid werden in 1 mL einer 1:1-Mischung aus THF/DMF gelöst. Es werden 17 mg (0.21 mmol) Isopropylisocyanat zugegeben und über Nacht bei 60°C gerührt. Das Solvens wird unter reduziertem Druck entfernt und das Rohprodukt mittels präparativer HPLC gereinigt. Es werden 23 mg (52% d.Th.) der Titelverbindung erhalten.
HPLC (Methode E): Rₜ = 4.13 min.
MS (ESIpos): *m*/*z* = 422 (M+H)⁺.

### Beispiel 25

### (4'-{[(3R)-1-Azabicyclo[2.2.2]oct-3-ylcarbonyl]amino}-1,1'-biphenyl-4-yl)methylethylcarbamat

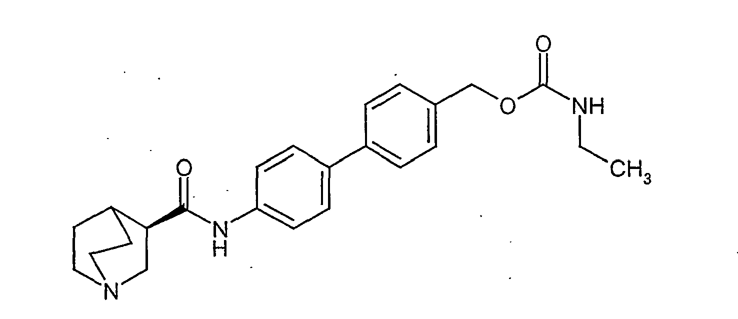

35 mg (0.10 mmol) (3*R*)-N-[4'-(Hydroxymethyl)-1,1'-biphenyl-4-yl]-1-azabicyclo-[2.2.2]octan-3-carboxamid werden in 1 mL einer 1:1-Mischung aus THF/DMF gelöst. Es werden 17 mg (0.21 mmol) Ethylisocyanat zugegeben und über Nacht bei 60°C gerührt. Das Solvens wird unter reduziertem Druck entfernt und das Rohprodukt mittels präparativer HPLC gereinigt. Es werden 24 mg (55% d.Th.) der Titelverbindung erhalten.
HPLC (Methode E): Rₜ = 3.97 min.
MS (ESIpos): *m*/*z* = 408 (M+H)⁺.

### Allgemeine Vorschrift zur Synthese der Ausführungsbeispiele 26-35:

32.3 mg (0.1 mmol) 2-(1-Azabicyclo[2.2.2]oct-3-yl)-N-(4-bromphenyl)acetamid-Hydrochlorid, 0.1 mmol der entsprechenden Boronsäure, 21.2 mg (0.2 mmol) Natriumcarbonat und 3.7 mg (0.01 mmol) Tetrakis(triphenylphosphin)palladium(0) werden in 0.5 mL Dioxan und 0.1 mL Wasser über Nacht auf 80°C erhitzt. Es wird mit DMSO verdünnt, filtriert und mittels präparativer HPLC aufgereinigt. Die Produkt-Fraktionen werden mit 2 N Salzsäure versetzt und im Vakuum eingeengt.

| **Bsp.-Nr.** | **Struktur** | **LC-MS (Methode F): [M+H]⁺ (freie Base)** |
|---|---|---|
| 26 | 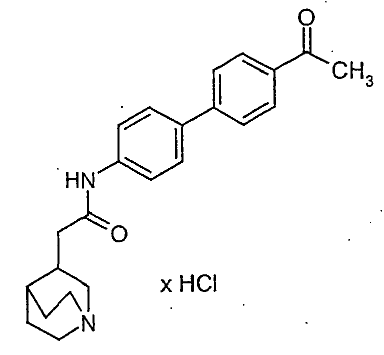 | 363 |
| 27 | 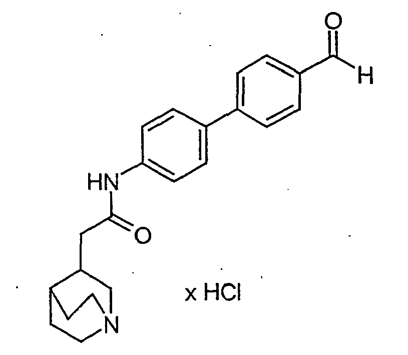 | 349 |
| 28 | 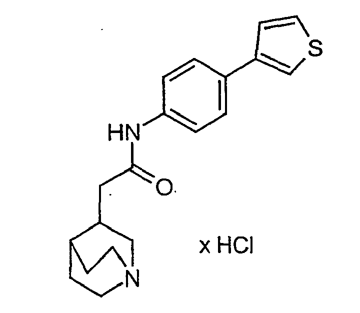 | 327 |
| 29 | 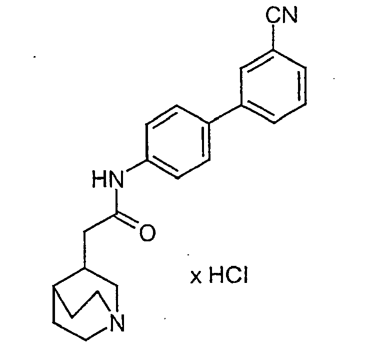 | 346 |
| 30 | 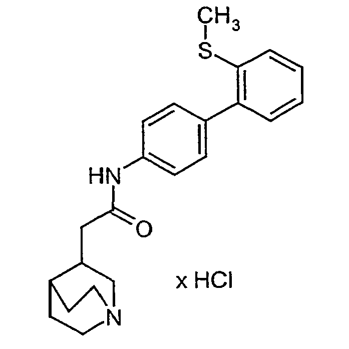 | 367 |
| 31 | 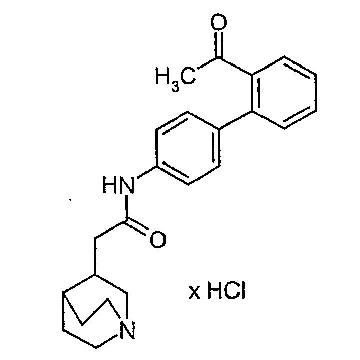 | 363 |
| 32 | 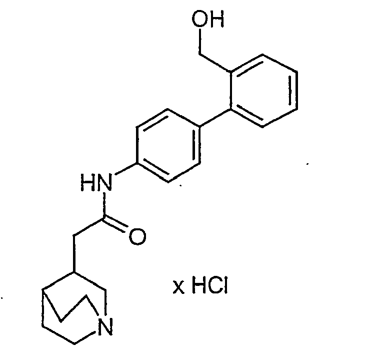 | 351 |
| 33 | 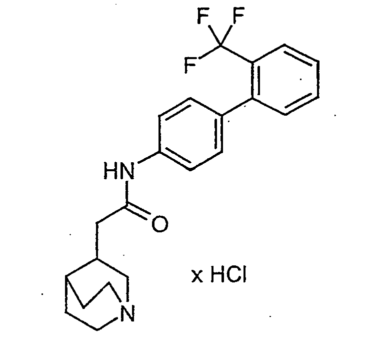 | 389 |
| 34 | 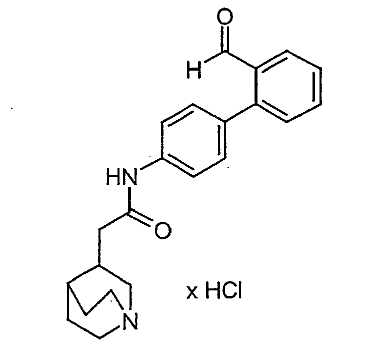 | 335 |
| 35 | 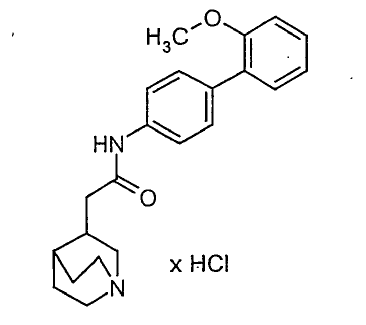 | 351 |

## Patentansprüche

1. Verbindungen der allgemeinen Formel (I) 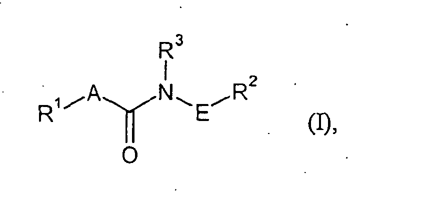 in welcher
R¹ für einen 1-Aza-blcyclo[m.n.p]alkyl-Rest mit 7 bis 11 Ringatomen steht,
worin m und n unabhängig voneinander 2 oder 3 bedeuten,
worin p 1, 2 oder 3 bedeutet,
und wobei der Bicycloalkylrest gegebenenfalls durch (C₁-C₆)-Alkyl substituiert ist,
A für eine Bindung, Methylen, Ethylen oder Propylen steht,
E für zweibindiges, 5- bis 6-gliedriges Heteroaryl oder Benzoldiyl steht, wobei Heteroaryl und Benzoldiyl gegebenenfalls durch Reste ausgewählt aus der Gruppe Halogen, Cyano, Trifluormethyl, Trifluormethoxy und (C₁-C₆)-Alkyl substituiert sind,
R² für 5- bis 6-gliedriges Heteroaryl oder Phenyl steht, wobei Heteroaryl und Phenyl gegebenenfalls durch Reste ausgewählt aus der Gruppe Halogen, 5- bis 6-gliedriges Heterocyclyl, -CO-NR⁴R⁵, -CO-OR⁶, -NR⁷R⁸, -NR⁹-CO-R¹⁰, -COR¹³, Cyano, Trifluormethyl, Trifluormethoxy, Nitro, gegebenenfalls durch Hydroxyl, Amino, -NH-CO-R¹¹, -O-CO-NHR¹⁴, Halogen oder Cyano substituiertes (C₁-C₆)-Alkyl, (C₁-C₆)-Alkoxy und (C₁-C₆)-Alkylthio substituiert sind,
worin R⁴, R⁵, R⁶, R⁷, R⁸, R⁹, R¹⁰ R¹¹, R¹³ und R¹⁴ unabhängig voneinander Wasserstoff, (C₁-C₆)-Alkyl, Phenyl oder Benzyl bedeuten,
und
R³ für Wasserstoff oder (C₁-C₆)-Alkyl steht,
und deren Salze, Solvate und Solvate der Salze.

2. Verbindungen nach Anspruch 1, in welcher
R¹ für einen 1-Aza-bicyclo[m.n.p]alkyl-Rest mit 7 bis 11 Ringatomen steht,
worin m und n unabhängig voneinander 2 oder 3 bedeuten,
worin p 1, 2 oder 3 bedeutet,
und wobei der Bicycloalkylrest gegebenenfalls durch (C₁-C₆)-Alkyl substituiert ist,
A für eine Bindung, Methylen, Ethylen oder Propylen steht,
E für zweibindiges, 5- bis 6-gliedriges Heteroaryl oder Benzoldiyl steht, wobei Heteroaryl und Benzoldiyl gegebenenfalls durch Reste ausgewählt aus der Gruppe Halogen, Cyario, Trifluormethyl, Trifluormethoxy und (C₁-C₆)-Alkyl substituiert sind,
R² für 5- bis 6-gliedriges Heteroaryl oder Phenyl steht, wobei Heteroaryl und Phenyl gegebenenfalls durch Reste ausgewählt aus der Gruppe Halogen, Formyl, -CO-NR⁴R⁵ -CO-OR⁶, -NR⁷R⁸, -NR⁹-CO-R¹⁰, Cyano, Trifluormethyl, Trifluormethoxy, Nitro, gegebenenfalls durch Hydroxyl, Amino, -NH-CO-R¹¹ oder Cyano substituiertes (C₁-C₆)-Alkyl, (C₁-C₆)-Alkoxy und (C₁-C₆)-Alkylthio substituiert sind, worin R⁴, R⁵, R⁶, R⁷, R⁸, R⁹, R¹⁰ und R¹¹ unabhängig voneinander Wasserstoff, (C₁-C₆)-Alkyl, Phenyl oder Benzyl bedeuten,
und
R³ für Wasserstoff oder (C₁-C₆)-Alkyl steht,
und deren Salze, Solvate und Solvate der Salze.

3. Verbindungen nach Anspruch 1, wobei
R¹ für 1-Aza-bicyclo[2.2.2]oct-3-yl steht,
und A, E, R² und R³ die in Anspruch 1 angegebenen Bedeutungen haben.

4. Verbindungen nach Ansprüchen 1, 2 oder 3, wobei
R¹ für 1-Aza-bicyclo[2.2.2]oct-3-yl steht,
A für eine Bindung oder Methylen steht,
E für Benzoldiyl, das gegebenenfalls durch einen Rest ausgewählt aus der Gruppe Fluor, Chlor, Cyano, Methyl und Trifluormethyl substituiert ist, steht,
R² für Thienyl oder Phenyl steht, wobei die Ringe gegebenenfalls mit bis zu 2 Resten ausgewählt aus der Gruppe Halogen, Cyano, Trifluormethyl, Trifluormethoxy, Nitro, Morpholinyl, (C₁-C₄)-Alkyl, (C₁-C₄)-Alkoxy, (C₁-C₄)-Alkylthio, -CO-NR⁴R⁵, -CO-OR⁶, -NR⁹-CO-R¹⁰ und -CO-R¹³ substituiert sind,
wobei (C₁-C₄)-Alkyl gegebenenfalls mit Hydroxy, Halogen und -O-CO-NHR¹⁴ substituiert ist,
wobei R⁴, R⁵, R⁶, R⁹, R¹⁰, R¹³ und R¹⁴ unabhängig voneinander für Wasserstoff oder (C₁-C₄)-Alkyl stehen,
und
R³ für Wasserstoff steht,
sowie deren Salze, Solvate und Solvate der Salze.

5. Verbindungen nach Ansprüchen 1, 2, 3 oder 4, wobei
R¹ für 1-Aza-bicyclo[2.2.2]oct-3-yl steht,
A für eine Bindung steht,
E für Benzoldiyl, das gegebenenfalls durch 1 bis 3 Reste ausgewählt aus der Gruppe Fluor, Chlor, Cyano, Methyl und Trifluormethyl substituiert ist, steht,
R² für Thienyl oder Phenyl steht, wobei die Ringe gegebenenfalls durch 1 bis 3 Reste ausgewählt aus der Gruppe Halogen, Cyano, Trifluormethyl, Trifluormethoxy, Nitro, (C₁-C₄)-Alkyl, Hydroxymethyl, und (C₁-C₄)-Alkoxy substituiert sind,
und
R³ für Wasserstoff steht.

6. Verfahren zur Herstellung von Verbindungen nach Ansprüchen 1 bis 5, **dadurch gekennzeichnet, dass** man
[A] Verbindungen der allgemeinen Formel (II) 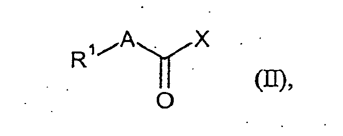 in welcher
R¹ und A die in Anspruch 1 angegebenen Bedeutungen haben, und
X für Hydroxy oder eine geeignete Abgangsgruppe wie beispielsweise Chlor oder Pentafluorphenoxy steht,
mit einer Verbindung der allgemeinen Formel (III)
R³-NH-E-R² (III),
in welcher R², R³ und E die in Anspruch 1 angegebenen Bedeutungen haben, umsetzt,
oder
[B] Verbindungen der allgemeinen Formel (V) 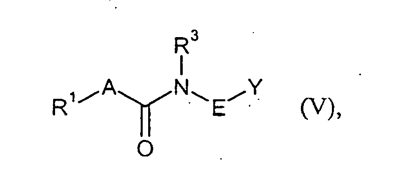
in welcher
Y für eine geeignete Abgangsgruppe wie beispielsweise Triflat oder Halogen, vorzugsweise Brom oder Iod, steht, und
R¹, R³, A und E die in Anspruch 1 angegebenen Bedeutungen haben,
mit Verbindungen der allgemeinen Formel (VI) 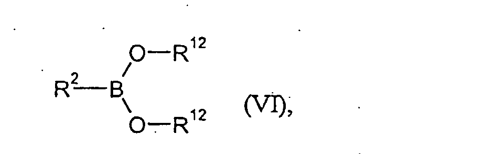 in welcher
R² die oben angegebenen Bedeutungen hat, und
R¹² für Wasserstoff oder Methyl steht oder beide Reste zusammen eine CH₂CH₂- oder C(CH₃)₂-C(CH₃)₂-Brücke bilden,
in einem inerten Lösungsmittel in Gegenwart eines geeigneten Katalysators und in Gegenwart einer Base umsetzt und die resultierenden Verbindungen der Formel (I) gegebenenfalls mit den entsprechenden (i) Lösungsmitteln und/oder (ii) Basen oder Säuren zu ihren Solvaten, Salzen und/oder Solvaten der Salze umsetzt.

7. Verbindungen nach einem der Ansprüche 1 bis 5 zur Behandlung und/oder Prophylaxe von Krankheiten.

8. Arzneimittel enthaltend mindestens eine der Verbindungen nach einem der Ansprüche 1 bis 5 in Zusammenmischung mit mindestens einem pharmazeutisch verträglichen, im Wesentlichen nichtgiftigen Träger oder Exzipienten.

9. Verwendung von Verbindungen nach einem der Ansprüche 1 bis 5 zur Herstellung eines Arzneimittels zur Verbesserung der Wahrnehmung, Konzentrationsleistung, Lernleistung und/oder Gedächtnisleistung.

10. Verwendung von Verbindungen nach einem der Ansprüche 1 bis 5 zur Herstellung eines Arzneimittels zur Behandlung und/oder Prophylaxe von Störungen der Wahrnehmung, Konzentrationsleistung, Lernleistung und/oder Gedächtnisleistung.

11. Arzneimittel nach Anspruch 8 zur Behandlung und/oder Prophylaxe von Störungen der Wahrnehmung, Konzentrationsleistung, Lernleistung und/oder Gedächtnisleistung.

## Claims

1. Compounds of the formula (I) 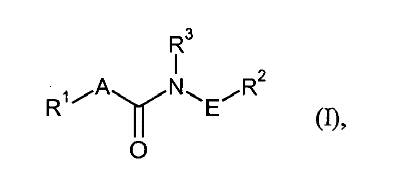 in which
R¹ is a 1-azabicyclo[m.n.p]alkyl radical having 7 to 11 ring atoms,
in which m and n are independently of one another 2 or 3,
in which p is 1, 2 or 3,
and where the bicycloalkyl radical is optionally substituted by (C₁-C₆)-alkyl,
A is a bond, methylene, ethylene or propylene,
E is divalent, 5- to 6-membered heteroaryl or benzenediyl, where heteroaryl and benzenediyl are optionally substituted by radicals selected from the group of halogen, cyano, trifluoromethyl, trifluoromethoxy and (C₁-C₆)-alkyl,
R² is 5- to 6-membered heteroaryl or phenyl, where heteroaryl and phenyl are optionally substituted by radicals selected from the group of halogen, 5- to 6-membered heterocyclyl, -CO-NR⁴R⁵, -CO-OR⁶, -NR⁷R⁸, -NR⁹-CO-R¹⁰, -COR¹³, cyano, trifluoromethyl, trifluoromethoxy, nitro, optionally hydroxyl-, amino-, -NH-CO-R¹¹-, -O-CO-NHR¹⁴-, halogen- or cyano- substituted (C₁-C₆)-alkyl, (C₁-C₆)-alkoxy and (C₁-C₆)-alkylthio,
in which R⁴, R⁵, R⁶, R⁷, R⁸, R⁹, R¹⁰, R¹¹, R¹³ and R¹⁴ are independently of one another hydrogen, (C₁-C₆)-alkyl, phenyl or benzyl,
and
R³ is hydrogen or (C₁-C₆)-alkyl,
and the salts, solvates and solvates of the salts thereof.

2. Compounds according to Claim 1, in which
R¹ is a 1-azabicyclo[m.n.p]alkyl radical having 7 to 11 ring atoms,
in which m and n are independently of one another 2 or 3,
in which p is 1, 2 or 3,
and where the bicycloalkyl radical is optionally substituted by (C₁-C₆)-alkyl,
A is a bond, methylene, ethylene or propylene,
E is divalent, 5- to 6-membered heteroaryl or benzenediyl, where heteroaryl and benzenediyl are optionally substituted by radicals selected from the group of halogen, cyano, trifluoromethyl, trifluoromethoxy and (C₁-C₆)-alkyl,
R² is 5- to 6-membered heteroaryl or phenyl, where heteroaryl and phenyl are optionally substituted by radicals selected from the group of halogen, formyl, -CO-NR⁴R⁵, -CO-OR⁶, -NR⁷R⁸, -NR⁹-CO-R¹⁰, cyano, trifluoromethyl, trifluoromethoxy, nitro, optionally hydroxyl-, amino-, -NH-CO-R¹¹- or cyano-substituted (C₁-C₆)-alkyl, (C₁-C₆)-alkoxy and (C₁-C₆)-alkylthio,
in which R⁴, R⁵, R⁶, R⁷, R⁸, R⁹, R¹⁰ and R¹¹ are independently of one another hydrogen, (C₁-C₆)-alkyl, phenyl or benzyl,
and
R³ is hydrogen or (C₁-C₆)-alkyl,
and the salts, solvates and solvates of the salts thereof.

3. Compounds according to Claim 1, where
R¹ is 1-azabicyclo[2.2.2]oct-3-yl,
and A, E, R² and R³ have the meanings indicated in claim 1.

4. Compounds according to Claims 1, 2 or 3, where
R¹ is 1-azabicyclo[2.2.2]oct-3-yl,
A is a bond or methylene,
E is benzenediyl which is optionally substituted by a radical selected from the group of fluorine, chlorine, cyano, methyl and trifluoromethyl,
R² is thienyl or phenyl, where the rings are optionally substituted by up to 2 radicals selected from the group of halogen, cyano, trifluoromethyl, trifluoromethoxy, nitro, morpholinyl, (C₁-C₄)-alkyl, (C₁-C₄)-alkoxy, (C₁-C₄)-alkylthio, -CO-NR⁴R⁵, -CO-OR⁶, -NR⁹-CO-R¹⁰ and -CO-R¹³,
where (C₁-C₄)-alkyl is optionally substituted by hydroxy, halogen and -O-CO-NHR¹⁴,
where R⁴, R⁵, R⁶, R⁹, R¹⁰, R¹³ and R¹⁴ are independently of one another hydrogen or (C₁-C₄)-alkyl,
and
R³ is hydrogen, and the salts, solvates and solvates of the salts thereof.

5. Compounds according to Claims 1, 2, 3 or 4, where
R¹ is 1-azabicyclo[2.2.2]oct-3-yl,
A is a bond,
E is benzenediyl which is optionally substituted by 1 to 3 radicals selected from the group of fluorine, chlorine, cyano, methyl and trifluoromethyl,
R² is thienyl or phenyl, where the rings are optionally substituted by 1 to 3 radicals selected from the group of halogen, cyano, trifluoromethyl, trifluoromethoxy, nitro, (C₁-C₄)-alkyl, hydroxymethyl, and (C₁-C₄)-alkoxy,
and
R³ is hydrogen.

6. Process for preparing compounds according to Claims 1 to 5, **characterized in that**
[A] compounds of the general formula (II) 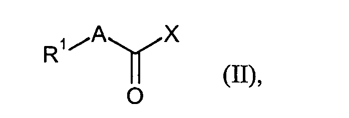 in which
R¹ and A have the meanings indicated in Claim 1, and
X is hydroxy or a suitable leaving group such as, for example, chlorine or pentafluorophenoxy,
are reacted with a compound of the general formula (III)
R³-NH-E-R² (III),
in which R², R³ and E have the meanings indicated in Claim 1, or
[B] compounds of the general formula (V) 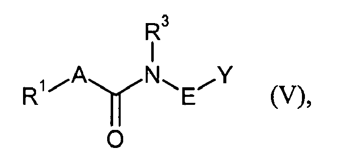 in which
Y is a suitable leaving group such as, for example, triflate or halogen, preferably bromine or iodine, and
R¹, R³, A and E have the meanings indicated in Claim 1,
are reacted with compounds of the general formula (VI) 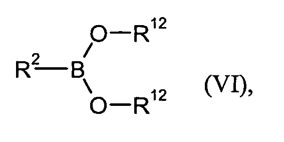 in which
R² has the meanings indicated above, and
R¹² is hydrogen or methyl, or the two radicals together form a CH₂CH₂ or C(CH₃)₂-C(CH₃)₂ bridge,
in an inert solvent in the presence of a suitable catalyst and in the presence of a base, and the resulting compounds of the formula (I) are where appropriate converted with the appropriate (i) solvents and/or (ii) bases or acids into the solvates, salts and/or solvates of the salts thereof.

7. Compounds according to any of Claims 1 to 5 for the treatment and/or prophylaxis of diseases.

8. Medicament comprising at least one of the compounds according to any of Claims 1 to 5 mixed with at least one pharmaceutically acceptable, essentially nontoxic carrier or excipient.

9. Use of compounds according to any of Claims 1 to 5 for producing a medicament for improving perception, concentration, learning and/or memory.

10. Use of compounds according to any of Claims 1 to 5 for producing a medicament for the treatment and/or prophylaxis of impairments of perception, concentration, learning and/or memory.

11. Medicament according to Claim 8 for the treatment and/or prophylaxis of impairments of perception, concentration, learning and/or memory.

## Revendications

1. Composés de formule générale (I) 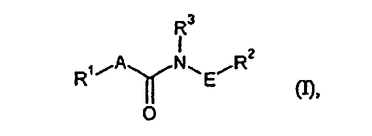 dans laquelle
R¹ représente un reste 1-aza-bicyclo[m.n.p]alkyle à noyau de 7 à 11 atomes,
où m et n ont indépendamment l'un de l'autre la valeur 2 ou 3,
où p a la valeur 1, 2 ou 3,
et le reste bicycloalkyle est éventuellement substitué par un radical alkyle en C₁ à C₆,
A représente une liaison, un groupe méthylène, éthylène ou propylène,
E représente un groupe hétéroaryle pentagonal ou hexagonal ou benzènediyle à deux liaisons, les groupes hétéroaryle et benzènediyle étant éventuellement substitués par des restes choisis dans le groupe de restes halogéno, cyano, trifluorométhyle, trifluorométhoxy et alkyle en C₁ à C₆,
R² représente un groupe hétéroaryle pentagonal ou hexagonal
ou un groupe phényle, les groupes hétéroaryle et phényle étant éventuellement substitués par des restes choisis dans le groupe des restes halogéno, hétérocyclyle pentagonal ou hexagonal, -CO-NR⁴R⁵, -CO-OR⁶, -NR⁷R⁸, -NR⁹-CO-R¹⁰, -COR¹³, cyano, trifluorométhyle, trifluorométhoxy, nitro, alkyle en C₁ à C₆ éventuellement substitué par un radical hydroxyle, amino, -NH-CO-R¹¹, -O-CO-NHR¹⁴, halogéno ou cyano, alkoxy en C₁ à C₆ et alkylthio en C₁ à C₆,
R⁴, R⁵, R⁶, R⁷, R⁸, R⁹, R¹⁰, R¹¹, R¹³ et R¹⁴ représentant, indépendamment les uns des autres, l'hydrogène, un reste alkyle en C₁ à C₆, phényle ou benzyle,
et
R³ représente l'hydrogène ou un reste alkyle en C₁ à C₆, et leurs sels, leurs produits de solvatation et les produits de solvatation des sels.

2. Composés suivant la revendication 1, dans lesquels
R¹ représente un reste 1-aza-bicyclo[m.n.p]alkyle à noyau de 7 à 11 atomes,
où m et n représentent indépendamment l'un de l'autre le nombre 2 ou 3,
où p représente le nombre 1, 2 ou 3,
et le reste bicycloalkyle est éventuellement substitué par un radical alkyle en C₁ à C₆,
A représente une liaison, un groupe méthylène, éthylène ou propylène,
E représente un groupe hétéroaryle pentagonal ou hexagonal ou benzènediyle à deux liaisons, les groupes hétéroaryle et benzènediyle étant éventuellement substitués par des restes choisis dans le groupe des restes halogéno, cyano, trifluorométhyle, trifluorométhoxy et alkyle en C₁ à C₆,
R² représente un reste hétéroaryle pentagonal ou hexagonal ou phényle, les groupes hétéroaryle et phényle étant éventuellement substitués par des restes choisis dans le groupe des restes halogéno, formyle, -CO-NR⁴R⁵, -CO-OR⁶, -NR⁷R⁸, -NR⁹-CO-R¹⁰, cyano, trifluorométhyle, trifluorométhoxy, nitro, alkyle en C₁ à C₆ éventuellement substitué par un radical hydroxyle, amino, -NH-CO-R¹¹ ou cyano, alkoxy en C₁ à C₆ ou alkylthio en C₁ à C₆,
R⁴, R⁵, R⁶, R⁷, R⁸, R⁹, R¹⁰ et R¹¹ représentant indépendamment les uns des autres l'hydrogène, un reste alkyle en C₁ à C₆, phényle ou benzyle,
et
R³ représente l'hydrogène ou un reste alkyle en C₁ à C₆,
et leurs sels, produits de solvatation et les produits de solvatation des sels.

3. Composés suivant la revendication 1, dans lesquels
R¹ représente un reste 1-aza-bicyclo[2.2.2]oct-3-yle,
et A, E, R² et R³ ont les définitions indiquées dans la revendication 1.

4. Composés suivant les revendications 1, 2 ou 3, dans lesquels
R¹ représente un reste 1-aza-bicyclo[2.2.2]oct-3-yle,
A représente une liaison ou un groupe méthylène,
E représente un groupe benzènediyle qui est éventuellement substitué par un reste choisi dans le groupe des restes fluoro, chloro, cyano, méthyle et trifluorométhyle,
R² représente un groupe thiényle ou phényle, les noyaux étant éventuellement substitués avec jusqu'à deux restes choisis dans le groupe des restes halogéno, cyano, trifluorométhyle, trifluorométhoxy, nitro, morpholinyle, alkyle en C₁ à C₄, alkoxy en C₁ à C₄, alkylthio en C₁ à C₄, -CO-NR⁴R⁵, -CO-OR⁶, -NR⁹-CO-R¹⁰ et -CO-R¹³,
le reste alkyle en C₁ à C₄ étant éventuellement substitué avec un radical hydroxy, halogéno et -O-CO-NHR¹⁴,
R⁴, R⁵, R⁶, R⁹, R¹⁰, R¹³ et R¹⁴ représentant indépendamment les uns des autres l'hydrogène ou un reste alkyle en C₁ à C₄,
et
R³ représente l'hydrogène,
ainsi que leurs sels, leurs produits de solvatation et les produits de solvatation des sels.

5. Composés suivant les revendications 1, 2, 3 ou 4,
dans lesquels
R¹ représente un reste 1-aza-bicyclo[2.2.2]oct-3-yle,
A représente une liaison,
E représente un groupe benzènediyle qui est éventuellement substitué par 1 à 3 restes choisis dans le groupe des restes fluoro, chloro, cyano, méthyle et trifluorométhyle,
R² représente un groupe thiényle ou phényle, les noyaux étant éventuellement substitués par 1 à 3 restes choisis dans le groupe des restes halogéno, cyano, trifluorométhyle, trifluorométhoxy, nitro, alkyle en C₁ à C₄, hydroxyméthyle et alkoxy en C₁ à C₄,
et
R³ représente l'hydrogène.

6. Procédé de production de composés suivant les revendications 1 à 5, **caractérisé en ce que**
[A] on fait réagir des composés de formule générale (II) 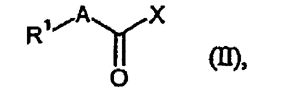 dans laquelle
R¹ et A ont les définitions indiquées dans la revendication 1, et
X représente un groupe hydroxy ou un groupe partant convenable tel que, par exemple, un groupe chloro ou pentafluorophénoxy,
avec un composé de formule générale (III)
**R³-NH-E-R²** **(III),**
dans laquelle R², R³ et E ont les définitions indiquées dans la revendication 1,
ou bien
[B] on fait réagir des composés de formule générale (V) 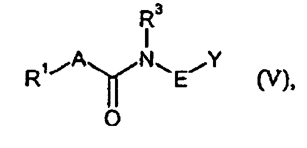 dans laquelle
Y représente ungroupe partant convenable tel que, par exemple, le groupe triflate ou un halogène, avantageusement le brome ou l'iode, et
R¹, R³, A et E ont les définitions indiquées dans la revendication 1,
avec des composés de formule générale (VI) 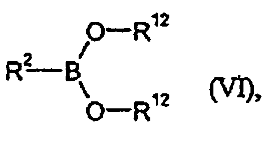 dans laquelle
R² a les définitions indiquées ci-dessus, et
R¹² représente l'hydrogène ou un reste méthyle, ou bien les deux restes forment conjointement un pont CH₂CH₂- ou C (CH₃)₂-C(CH₃)₂-,
dans un solvant inerte, en présence d'un catalyseur convenable et en présence d'une base, et on fait réagir éventuellement les composés de formule (I) résultants avec (i) les solvants correspondants et/ou (ii) les bases ou les acides correspondants pour former leurs produits de solvatation, leurs sels et/ou les produits de solvatation des sels.

7. Composés suivant l'une des revendications 1 à 5, destinés au traitement et/ou à la prophylaxie de maladies.

8. Médicament contenant au moins l'un des composés suivant l'une des revendications 1 à 5, en mélange avec au moins un support ou excipient pharmaceutiquement acceptable, principalement non toxique.

9. Utilisation de composés suivant l'une des revendications 1 à 5, pour la préparation d'un médicament destiné à améliorer la perception, la capacité de concentration, la capacité d'apprentissage et/ou la capacité de mémoire.

10. Utilisation de composés suivant l'une des revendications 1 à 5, pour la préparation d'un médicament destiné au traitement et/ou à la prophylaxie de troubles de la perception, de la capacité de concentration, de la capacité d'apprentissage et/ou de la capacité de mémoire.

11. Médicament suivant la revendication 8, destiné au traitement et/ou à la prophylaxie de troubles de la perception, de la capacité de concentration, de la capacité d'apprentissage et/ou de la capacité de mémoire.
